# EUROPEAN PATENT SPECIFICATION

(11) **EP 2 364 999 B1**
(45) Date of publication and mention of the grant of the patent: **14.10.2015**
(21) Application number: 10172263.5
(22) Date of filing: 28.06.2002
(51) Int. Cl.: C07K 16/46, C12N 15/13, C12N 15/79, C12N 5/10, G01N 33/68, G01N 33/577, G01N 33/542

(54) **Dual-specific ligand and its use**
Doppelspezifischer Ligand und dessen Verwendung
Ligand à double spécificité et son utilisation

(30) Priority: 28.06.2001 GB 0115841
(43) Date of publication of application: 14.09.2011
(62) Divisional of application: 02748987.1
(73) Proprietor: Domantis Limited, Brentford, Middlesex TW8 9GS (GB)
(72) Inventor: Winter, Greg, Cambridge CB2 2QH (GB); Ignatovich, Olga, Cambridge CB4 0WG (GB); Tomlinson, Ian, Cambridge CB4 0WG (GB)
(74) Representative: Furlong, Isla Jane

(56) References cited:
- EP-A1- 0 368 684
- WO-A-00/29004
- WO-A-90/05144
- WO-A-92/01787
- US-A- 5 989 830
- SMITH B J ET AL: "Prolonged in vivo residence times of antibody fragments associated with albumin", BIOCONJUGATE CHEMISTRY, ACS, WASHINGTON, DC, US, vol. 12, no. 5, 1 September 2001 (2001-09-01), pages 750-756, XP002270731, ISSN: 1043-1802, DOI: 10.1021/BC010003G
- MUYLDERMANS S: "SINGLE DOMAIN CAMEL ANTIBODIES: CURRENT STATUS", REVIEWS IN MOLECULAR BIOTECHNOLOGY, ELSEVIER, AMSTERDAM, NL, vol. 74, no. 4, 1 June 2001 (2001-06-01), pages 277-302, XP001057480, ISSN: 1389-0352, DOI: 10.1016/S1389-0352(01)00021-6
- HOOGENBOOM H: "Mix and match: Building manifold binding sites", NATURE BIOTECHNOLOGY, NATURE PUBLISHING, US, vol. 15, no. 2, 1 February 1997 (1997-02-01), pages 125-126, XP002084229, ISSN: 1087-0156
- MARTSEV S P ET AL: "Antiferritin single-chain antibody: a functional protein with incomplete folding?", FEBS LETTERS, ELSEVIER SCIENCE PUBLISHERS, AMSTERDAM, NL, vol. 441, no. 3, 28 December 1998 (1998-12-28), pages 458-462, XP004258983, ISSN: 0014-5793
- REITER Y ET AL: "An antibody single-domain phage display library of a native heavy chain variable region: Isolation of functional single-domain VH molecules with a unique interface", JOURNAL OF MOLECULAR BIOLOGY, LONDON, GB, vol. 290, no. 3, 16 July 1999 (1999-07-16) , pages 685-698, XP002165613, ISSN: 0022-2836

## Description

The present invention relates to a method for the preparation of dual-specific ligands comprising a first single immunoglobulin variable domain region binding to a first antigen or epitope, and a second complementary immunoglobulin single variable domain region binding to a second antigen or epitope. Dual-specific ligands and their uses are also described.

### Introduction

The antigen binding domain of an antibody comprises two separate regions: a heavy chain variable domain (V_{H}) and a light chain variable domain (V_{L}: which can be either V_{κ} or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H1 and H2), whilst the V_{H}, D and J_{H} segments combine to form the third antigen binding loop of the V_{H} domain (H3). The V_{L} gene is produced by the recombination of only two gene segments, V_{L} and J_{L}. In humans, there are approximately 40 functional V_{K} segments (Schäble and Zachau (1993) Biol. Chem. Hoppe-Seyler, 374: 1001), 31 functional V_{λ} segments (Williams et al. (1996) J. Mol. Biol., 264: 220; Kawasaki et al. (1997) Genome Res., 7: 250), 5 functional J_{κ} segments (Hieter et al. (1982) J. Biol. Chem., 257: 1516) and 4 functional J_{λ} segments (Vasicek and Leder (1990) J. Exp. Med., 172: 609), depending on the haplotype. The V_{L} segment encodes the region of the polypeptide chain which forms the first and second antigen binding loops of the V_{L} domain (L1 and L2). whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences *(*Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

Bispecific antibodies comprising complementary pairs of VH and VL regions are known in the art. These bispecific antibodies must comprise two pairs of VH and VLs, each VH/VL pair binding to a single antigen or epitope. Methods described involve hybrid hybridomas (Milstein & Cuello AC, Nature 305:537-40), minibodies (Hu et al., (1996) Cancer Res 56:3055-3061;), diabodies (Holliger et al., (1993) Proc. Natl. Acad. Sci. USA 90, 6444-6448; WO 94/13804), chelating recombinant antibodies (CRAbs; (Neri et al., (1995) J. Mol. Biol. 246, 367-373), biscFv (e.g. Atwell et al., (1996) Mol. Immunol. 33, 1301-1312), "knobs in holes" stabilised antibodies (Carter et al., (1997) Protein Sci. 6, 781-788). In each case each antibody species comprises two antigen-binding sites, each fashioned by a complementary pair of VH and VL domains. Each antibody is thereby able to bind to two different antigeus or epitopes at the same time, with the binding to EACH antigen or epitope mediated by a VH AND its complementary VL domain. Each of these techniques presents its particular disadvantages; for instance in the case of hybrid hybridomas, inactive VH/VL pairs can greatly reduce the fraction of bispecific IgG. Furthermore, most bispecific approaches rely on the association of the different VH/VL pairs or the association of VH and VL chains to recreate the two different VH/VL binding sites. It is therefore impossible to control the ratio of binding sites to each antigen or epitope in the assembled molecule and thus many of the assembled molecules will bind to one antigen or epitope but nor the other. In some cases it has been possible to engineer the heavy or light chains at the sub-unit interfaces (Carter *et al.,* 1997) in order to improve the number of molecules which have binding sites to both antigens or epitopes but this never results in all molecules having binding to both antigens or epitopes.

There is some evidence that two different antibody binding specificities might be incorporated into the same binding site, but these generally represent two or more specificities that correspond to structurally related antigens or epitopes or to antibodies that are broadly cross-reactive.. For example, cross-reactive antibodies have been described, usually where the two antigens are related in sequence and structure, such as hen egg white lysozyme and turkey lysozyme (McCafferty et al., WO 92/01047) or to free hapten and to hapten conjugated to carrier (Griffiths AD et al. EMBO J 1994 13:14 3245-60). In a further example, WO 02/02773 (Abbott Laboratories), published after the priority date of the present application, describes antibody molecules with "dual specificity". The antibody molecules referred to are antibodies raised or selected against multiple antigens, such that their specificity spans more than a single antigen. Each complementary V_{H}/V_{L} pair in the antibodies of WO 02/02773 specifies a single binding specificity for two or more structurally related antigens; the V_{H} and V_{L} domains in such complementary pairs do not each possess a separate specificity. The antibodies thus have a broad single specificity which encompasses two antigens, which are structurally related. Furthermore natural autoantibodies have been described that are polyreactive (Casali & Notkins, Ann. Rev. Immunol. 7, 515-531), reacting with at least two (usually more) different antigens or epitopes that are not structurally related. It has also been shown that selections of random peptide repertoires using phage display technology on a monoclonal antibody will identify a range of peptide sequences that fit the antigen binding site. Some of the sequences are highly related, fitting a consensus sequence, whereas others are very different and have been termed mimotopes (Lane & Stephen, Current Opinion in Immunology, 1993, 5, 268-271). It is therefore clear that the binding site of an antibody, comprising associated and complementary VH and VL domains, has the potential to bind to many different antigens from a large universe of known antigens. It is less clear how to create a binding site to two given antigens, particularly those which are not necessarily structurally related.

Protein engineering methods have been suggested that may have a bearing on this. For example it has also been proposed that a catalytic antibody could be created with a binding activity to a metal ion through one variable domain, and to a hapten (substrate) through contacts with the metal ion and a complementary variable domain (Barbas et al., 1993 Proc. Natl. Acad. Sci USA 90, 6385-6389). However in this case, the binding and catalysis of the substrate (first antigen) is proposed to require the binding of the metal ion (second antigen). Thus the binding to the VH/VL pairing relates to a single but multicomponent antigen.

Methods have been described for the creation of bispecific antibodies from camel antibody heavy chain single domains in which binding contacts for one antigen are created in one variable domain, and for a second antigen in a second variable domain. However the variable domains were not complementary. Thus a first heavy chain variable domain is selected against a first antigen, and a second heavy chain variable domain against a second antigen, and then both domains are linked together on the same chain to give a bispecific antibody fragment (Conrath et al., J. Biol. Chem. 270, 27589-27594). However the camel heavy chain single domains are unusual in that they are derived from natural camel antibodies which have no light chains, and indeed the heavy chain single domains are unable to associate with camel light chains to form complementary VH and VL pairs.

Single heavy chain variable domains have also been described, derived from natural antibodies which are normally associated with light chains (from monoclonal antibodies or from repertoires of domains EP-A-0368684). It was suggested to make bispecific antibody fragments by linking heavy chain variable domains of different specificity together (as described above). The disadvantage with this approach is that isolated antibody variable domains may have a hydrophobic interface that normally makes interactions with the light chain and is exposed to solvent and may be "sticky" allowing the single domain to bind to hydrophobic surfaces. Furthermore in this case the heavy chain variable domains would not be associated with complementary light chain variable domains and thus may be less stable and readily unfold (Worn & Pluckthun, 1998 Biochemistry 37, 13120-7).

Bryan et al., Bioconjugate Chem., 2001, 12, 750-756, discloses that the in vivo residence times of antibody fragments are prolonged when associated with albumin. The authors produce a bispecific F(ab')₂ which binds to TNF and rat serum albumin and has an increased in-vivo half-life compared to a construct not binding to albumin.

### Summary of the invention

The inventors have realised that it is desirable to make bispecific antibodies in which the binding of a first antigen or epitope does not necessarily facilitate the binding of a second antigen or epitope. They have also realised that the solution lies in creating binding contacts for the first antigen or epitope in one variable domain, and binding contacts for the second antigen or epitope in another variable domain, the domains being selected so that they are mutually complementary, and that further significant advantages over the bispecific antibodies of the prior art may be derived by bringing together complementary single variable domains of differing specificities; for example, a heavy chain variable domain that binds to a first antigen or epitope with a light chain variable domain that binds to a second antigen or epitope. Thus each VH/VL pair has two binding specificities. These combinations of domains are referred to as 'dual-specific' ligands.

The inventors have found that the use of complementary variable domains allows the two domain surfaces to pack together and be sequestered from the solvent. Furthermore the complementary domains are able to stabilise each other. In addition, it allows the creation of dual-specific IgG antibodies without the disadvantages of hybrid hybridomas previously discussed, or the need to engineer heavy or light chains at the sub-unit interfaces. The dual-specific ligands of the present invention have at least one VH/VL pair. A bispecific IgG according to this invention will therefore comprise two such pairs, one pair on each arm of the Y-shaped molecule. Unlike conventional bispecific antibodies or diabodies, therefore, where the ratio of chains used is determinative in the success of the preparation thereof and leads to practical difficulties, the dual specific ligands of the invention are free from issues of chain balance. Chain imbalance in conventional bi-specific antibodies results from the association of two different VL chains with two different VH chains, where VL chain 1 together with VH chain 1 is able to bind to antigen or epitope 1 and VL chain 2 together with VH chain 2 is able to bind to antigen or epitope 1 and the two correct pairings are in some way linked to one another. Thus, only when VL chain 1 is paired with VH chain 1 and VL chain 2 is paired with VH chain 2 in a single molecule is bi-specificity created. Such bi-specific molecules can be created in two different ways. Firstly, they can be created by association of two existing VH/VL pairings that each bind to a different antigen or epitope (for example, in a bi-specific IgG). In this case the VH/VL pairings must come all together in a 1:1 ratio in order to create a population of molecules all of which are bi-specific. This never occurs (even when complementary CH domain is enhanced by "knobs into holes" engineering) leading to a mixture of bi-specific molecules and molecules that are only able to bind to one antigen or epitope but not the other. The second way of creating a bi-specific antibody is by the simultaneous association of two different VH chain with two different VL chains (for example in a bi-specific diabody). In this case, although there tends to be a preference for VL chain 1 to pair with VH chain 1 and VL chain 2 to pair with VH chain 2 (which can be enhanced by "knobs into holes" engineering of the VL and VH domains), this paring is never achieved in all molecules, leading to a mixed formulation whereby incorrect pairings occur that are unable to bind to either antigen or epitope.

Bi-specific antibodies constructed according to the dual-specific ligand approach overcome all of these problems because the binding to antigen or epitope 1 resides within the VH or VL domain and the binding to antigen or epitope 2 resides with the complementary VL or VH domain, respectively. Since VH and VL domains pair on a 1:1 basis all VH/VL pairings will be bi-specific and thus all formats constructed using these VH/VL pairings (Fv, scFvs, Fabs, minibodies, IgGs etc) will have 100% bi-specific activity.

The present disclosure provides a method for producing a dual-specific ligand comprising a first single immunoglobulin variable domain having a first binding specificity and a complementary immunoglobulin single variable domain having a second binding specificity, the method comprising the steps of:
(a) selecting a first variable domain by its ability to bind to a first epitope,
(b) selecting a second variable region by its ability to bind to a second epitope,
(c) combining the variable regions; and
(d) selecting the dual-specific ligand by its ability to bind to said first and second epitopes.

In the context of the present invention, first and second "epitopes" are understood to be epitopes which are not the same and are not bound by a single monospecific ligand. They may be on different antigens or on the same antigen, but separated by a sufficient distance that they do not form a single entity that could be bound by a single mono-specific V_{H}/V_{L} binding pair of a conventional antibody. Experimentally, if both of the individual variable domains in single chain antibody form (domain antibodies or dAbs) are separately competed by a monospecific V_{H}/V_{L} ligand against two epitopes then those two epitopes are not sufficiently far apart to be considered separate epitopes according to the present invention.

The dual specific ligands of the invention do not include ligands as described in WO 02/02773. Thus, the ligands of the present invention do not comprise complementary V_{H}/V_{L} pairs which bind any one or more antigens or epitopes co-operatively. Instead, the ligands according to the invention comprise a V_{H}/V_{L} complementary pair, wherein the V domains have different specificities.

Moreover, the ligands according to the disclosure comprise V_{H}/V_{L} complementary pairs having different specificities for non-structurally related epitopes or antigens. Structurally related epitopes or antigens are epitopes or antigens which possess sufficient structural similarity to be bound by a conventional V_{H}/V_{L} complementary pair which acts in a co-operative manner to bind an antigen or epitope; in the case of structurally related epitopes, the epitopes are sufficiently similar in structure that they "fit" into the same binding pocket formed at the antigen binding site of the V_{H}/V_{L} dimer.

In a preferred embodiment of the disclosure each single variable domain may be selected for binding to its target antigen or epitope in the absence of a complementary variable region. In an alternative embodiment, the single variable domains may be selected for binding to its target antigen or epitope in the presence of a complementary variable region. Thus the first single variable domain may be selected in the presence of a third complementary variable domain, and the second variable domain may be selected in the presence of a fourth complementary variable domain. In this case the binding activity of first (or second) variable domain may not be evident except in the presence of the complementary third (or fourth) variable domain. The complementary third or fourth variable domain may be the natural cognate variable domain having the same specificity as the single domain being tested, or a non-cognate complementary domain — such as a "dummy" variable domain.

Advantageously, the single variable domains are derived from antibodies selected for binding activity against different antigens or epitopes.

Preferably, the dual specific ligand of the invention comprises only two complementary variable domains although several such ligands may be incorporated together into the same protein, for example two such ligands can be incorporated into an IgG or a multimeric immunoglobulin, such as IgM. Alternatively, in another embodiment a plurality of dual specific ligands are combined to form a multimer. For example, two different dual specific ligands are combined to create a tetra-specific molecule

Dual specific ligands may be combined into non-immunoglobulin multi-ligand structures to form multivalent complexes, which bind target molecules with increased avidity. In an example of such multimers, the V regions bind different epitopes on the same antigen, thereby providing superior avidity. In one embodiment multivalent complexes may be constructed on scaffold proteins, as described in WO0069907 (Medical Research Council), which are based for example on the ring structure of bacterial GroEL or to other chaperone polypeptides.

It will be appreciated by one skilled in the art that the light and heavy variable regions of a dual-specific ligand produced according to the method of the present disclosure may be on the same polypeptide chain, or alternatively, on different polypeptide chains. In the case that the variable regions are on different polypeptide chains, then they may be linked via a linker, generally a flexible linker (such as a polypeptide chain), a chemical linking group, or any other method known in the art.

The first and the second antigen binding domains may be associated either covalently or non-covalently. In the case that the domains are covalently associated, then the association may be mediated for example by disulphide bonds.

The first and the second antigens or epitopes are different. They may be, or be part of, polypeptides, proteins or nucleic acids, which may be naturally occurring or synthetic. One skilled in the art will appreciate that the choice is large and varied. They may be for instance human or animal proteins, , cytokines, cytokine receptors, enzymes co-factors for enzymes or DNA binding proteins. Suitable cytokines and growth factors include but are not limited to: ApoE, Apo-SAA, BDNF, Cardiotrophin-1, EGF, EGF receptor, ENA-78, Eotaxin, Eotaxin-2, Exodus-2, FGF-acidic, FGF-basic, fibroblast growth factor-10 (30). FLT3 ligand, Fractalkine (CX3C), GDNF, G-CSF, GM-CSF, GF-β1, insulin, IFN-y, IGF-I, IGF-II, II,-1α, IL-1β, IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α , Inhibin β, IP-10, keratinocyte growth factor-2 (KGF-2), KGF, Leptin, LIF, Lymphotactin, Mullerian inhibitory substance, monocyte colony inhibitory factor, monocyte attractant protein (30 *ibid*)*,* M-CSF, MDC (67 a.a.), MDC (69 a.a.), MCP-1 (MCAF), MCP-2, MCP-3, MCP-4, MDC (67 a.a.), MDC (69 a.a.), MIG, MIP-1α, MIP-1β, MIP-3α, MIP-3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF1α, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-γ, HCC1, 1-309, HER 1, HER 2, HER 3 and HER 4. Cytokine receptors include receptors for the foregoing cytokines. It will be appreciated that this list is by no means exhaustive. Where the dual specific ligand binds to two epitopes (on the same or different antigens), the antigen(s) may be selected from this list.

The antigens or epitopes may compete for binding to the dual-specific ligand, such that they may not both bind simultaneously. Alternatively, they may both bind simultaneously, such that the dual-specific ligand bridges the antigens or epitopes.

In one embodiment of the disclosure, the variable domains are derived from an antibody directed against the first and/or second antigen or epitope. In a preferred embodiment the variable domains are derived from a repertoire of single variable antibody domains.

In a second aspect, the present disclosure provides a dual-specific ligand comprising a first single immunoglobulin variable domain having a first binding specificity and a complementary immunoglobulin single variable domain having a second binding specificity.

Advantageously, the dual-specific ligand according to the second aspect of the invention is obtainable by the method of the first aspect of the present disclosure.

In a preferred embodiment of this aspect of the disclosure, the ligand comprises one single heavy chain variable domain of an antibody and one complementary single light chain variable domain of an antibody such that the two regions are capable of associating to form a complementary VH/VL pair.

A dual-specific ligand of this nature permits the two complementary variable region surfaces to pack together and be sequestered from the solvent and to help stabilise each other.

The invention is a dual-specific ligand comprising a first single immunoglobulin variable domain having a binding specificity to a first antigen which is human serum albumin (HSA) and a second complementary immunoglobulin single variable domain having a binding activity to a second antigen, wherein the first or second single variable domain is a V_{H} domain and the other complementary single variable domain is a V_{L} domain and a method for producing this ligand.

In a preferred embodiment, the dual specific ligand may be capable of binding the target molecule only on displacement of the half-life enhancing molecule or group. Thus, for example, a dual specific ligand is maintained in circulation in the bloodstream of a subject by a bulky molecule such as HSA. When a target molecule is encountered, competition between the binding domains of the dual specific ligand results in displacement of the HSA and binding of the target.

In a further aspect, the present invention provides one or more nucleic acid molecules encoding at least a dual-specific ligand as herein defined. The dual specific ligand may be encoded on a single nucleic acid molecule; alternatively, each complementary domain may be encoded by a separate nucleic acid molecule. Where the ligand is encoded by a single nucleic acid molecule, the complementary domains may be expressed as a fusion polypeptide, in the manner of a scFv molecule, or may be separately expressed and subsequently linked together, for example using chemical linking agents. Ligands expressed from separate nucleic acids will be linked together by appropriate means.

The nucleic acid may further encode a signal sequence for export of the polypeptides from a host cell upon expression and may be fused with a surface component of a filamentous bacteriophage particle (or other component of a selection display system) upon expression.

In a further aspect the present invention provides a vector comprising nucleic acid according to the present invention.

In a yet further aspect, the present invention provides a host cell transfected with a vector according to the present invention.

Expression from such a vector may be configured to produce, for example on the surface of a bacteriophage particle, variable domains for selection. This allows selection of displayed variable regions and thus selection of 'dual-specific ligands' using the method of the present invention.

The present disclosure further provides a kit comprising at least a dual-specific ligand according to the present invention.

Dual-Specific ligands according to the present invention comprise combinations of heavy and light chain domains. For example, the dual specific ligand may comprise a V_{H} domain and a V_{L} domain, which may be linked together in the form of an scFv. In addition, the ligands may comprise one or more C_{H} or C_{L} domains. For example, the ligands may comprise a C_{H}1 domain, C_{H}2 or C_{H}3 domain, and/or a C_{L} domain, Cµ1, Cµ2, Cµ3 or Cµ4 domains, or any combination thereof. A hinge region domain may also be included. Such combinations of domains may, for example, mimic natural antibodies, such as IgG or IgM, or fragments thereof, such as Fv, scFv, Fab or F(ab')₂ molecules. Other structures, such as a single arm of an IgG molecule comprising V_{H}, V_{L,} C_{H}1 and C_{L} domains, are envisaged.

In a preferred embodiment of the invention, the variable regions are selected from single domain V gene repertoires. Generally the repertoire of single antibody domains is displayed on the surface of filamentous bacteriophage. In a preferred embodiment each single antibody domain is selected by binding of a phage repertoire to antigen.

In a further aspect, the present invention provides a composition comprising a dual-specific ligand, obtainable by a method of the present invention, and a pharmaceutically acceptable carrier, diluent or excipient.

Moreover, the present disclosure provides a method for the treatment of disease using a 'dual-specific ligand' or a composition according to the present invention.

In a preferred embodiment of the disclosure the disease is cancer. For instance a 'bridging' dual specific ligand may be used to recruit cytotoxic T-cells to a cancer marker, or to bind to two different antigens or epitopes on the surface of a cancer cell, thereby increasing the affinity or specificity of binding to the cell surface. For a complete IgG, comprised of bridging dual specific ligands, the antibody would be capable of binding to four molecules of antigen or four different epitopes. Alternatively if the binding of one antigen or epitope displaces the other, such antibodies might be used to release a drug on binding of a cancer cell surface marker. Where the dual specific antibody is at least divalent, such as a dual specific IgG, multiple effectors may be delivered to the same cell, such as an anti-tumour drug and a cytotoxic T-cell marker.

In a further aspect, the present disclosure provides a method for the diagnosis, including diagnosis of disease using a dual-specific ligand, or a composition according to the present invention. Thus in general the binding of an analyte to a dual specific ligand may be exploited to displace an agent, which leads to the generation of a signal on displacement. For example, binding of analyte (second antigen) could displace an enzyme (first antigen) bound to the antibody providing the basis for an immunoassay, especially if the enzyme were held to the antibody through its active site.

### Brief Description of the Figures.

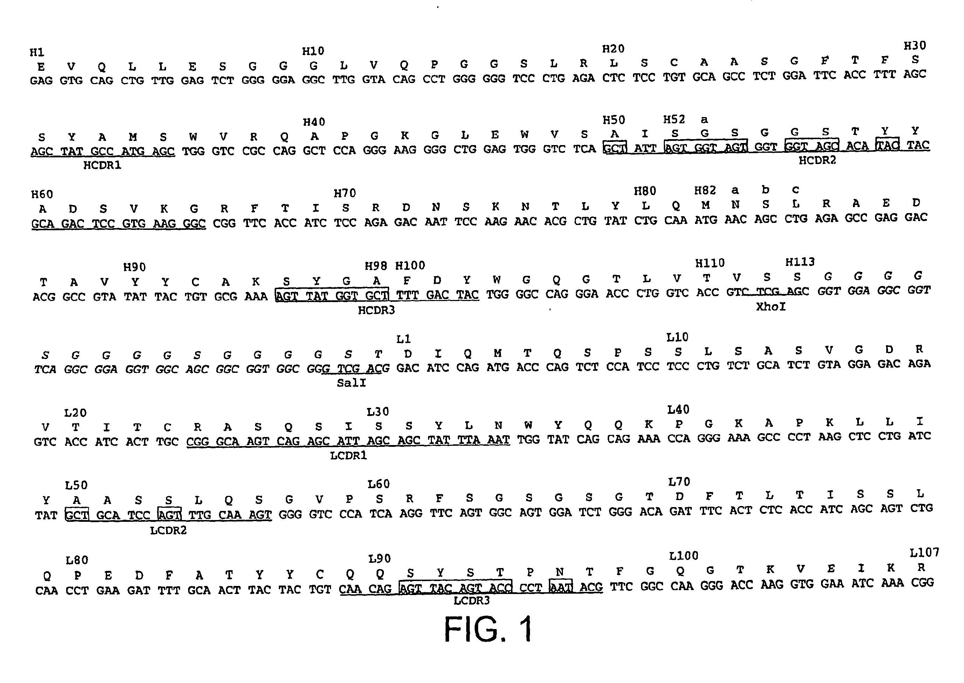

- Figure 1: shows the diversification of VH/HSA at positions H50, H52, H52a, H53, H55, H56, H58, H95, H96, H97, H98 (DVT or NNK encoded respectively) which are in the antigen binding site of VH HSA. The sequence of V_{K} is diversified at positions L50, L53.

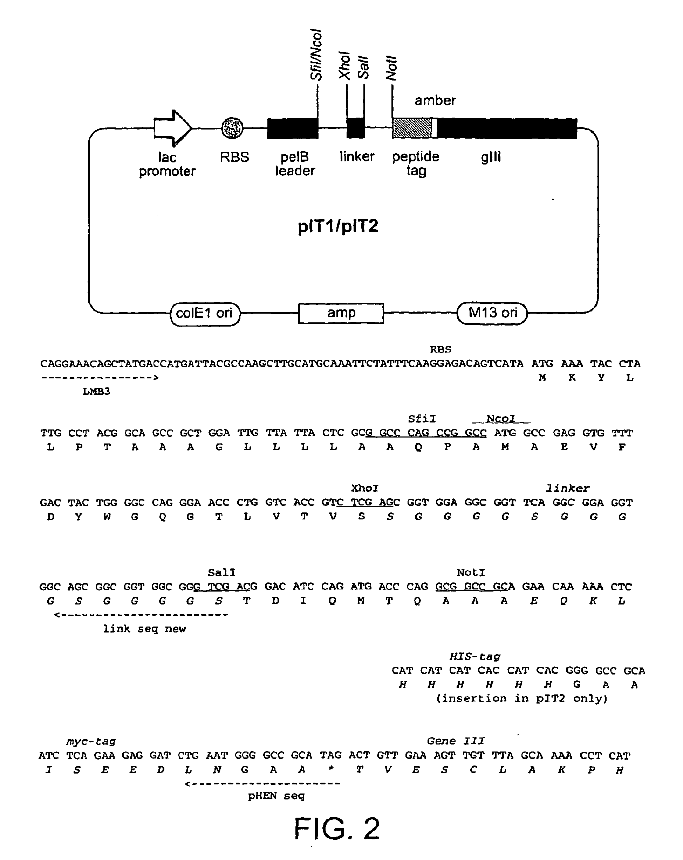

- Figure 2: shows Library 1: Germline V_{K}/DVT V_{H}, Library 2: Germline V_{K}/NNK V_{H}, Library 3: Germline V_{H}/DVT V_{K} Library 4: Germline V_{H}/NNK V_{K} In pIT2/ScFv format. These libraries were pre-selected for binding to generic ligands protein A and protein L so that the majority of the clones and selected libraries are functional. Libraries were selected on HSA (first round) and β-gal (second round) or HSA β-gal selection or on β-gal (first round) and HSA (second round) β-gal HSA selection. Soluble scFv from these clones of PCR are amplified in the sequence. One clone encoding a dual specific antibody K8 was chosen for further work.

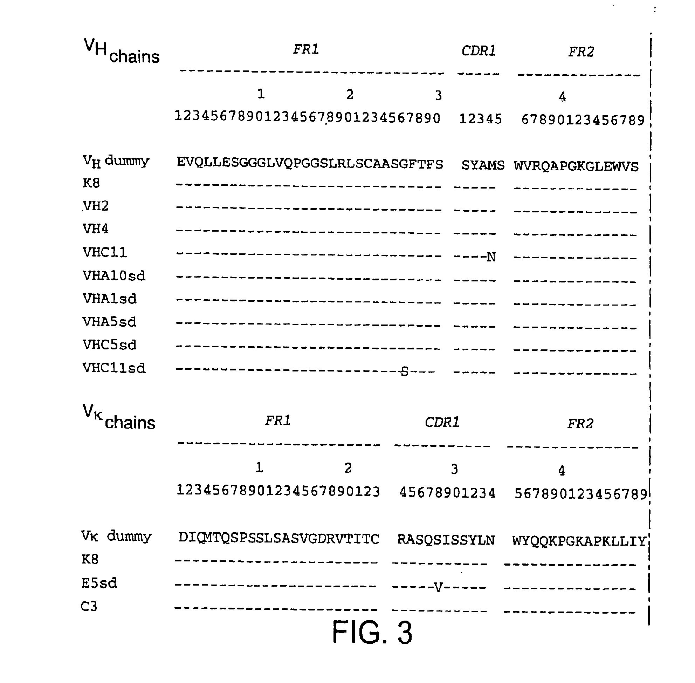

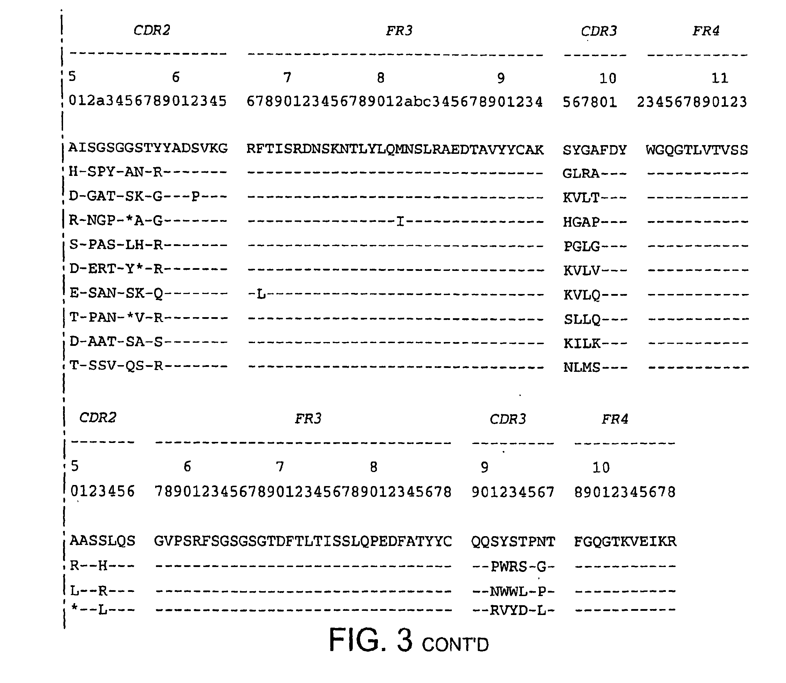

- Figure 3: shows an alignment of V_{H} chains and V_{K} chains.

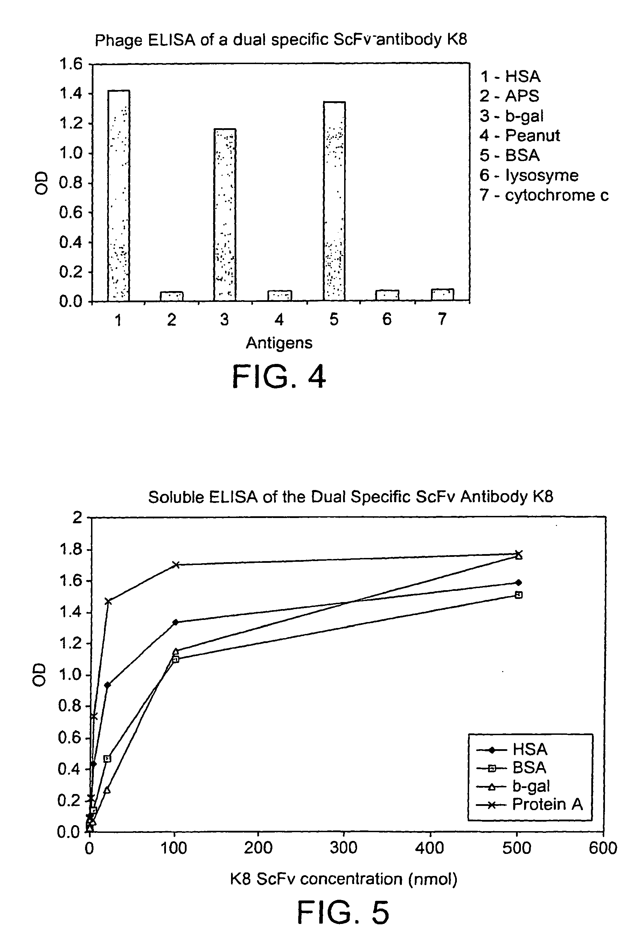

- Figure 4: shows the characterisation of the binding properties of the K8 antibody, the binding properties of the K8 antibody characterised by monoclonal faguliser, the dual specific K8 antibody was found to bind HSA and β -gal and displayed on the surface of the phage with absorbant signals greater than 1.0. No cross reactivity with other proteins was detected.
- Figure 5: shows soluble scFv ELISA performed using known concentrated and some of the K8 antibody fragment. A 96-well plate was coated with 100µg of HSA, BSA and β-gal at 10µg/ml and 100µg/ml of Protein A at 1µg/ml concentration. 50µg of the serial dilutions of the K8 scFv was applied and the bound antibody fragments were detected with Protein L-HRP. ELISA results confirm the dual specific nature of the K8 antibody.

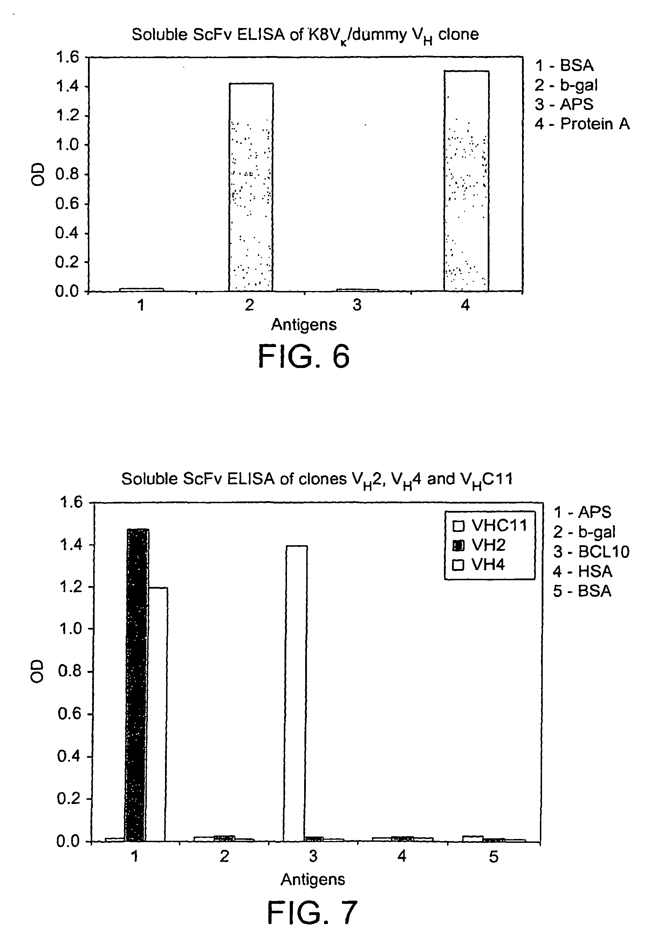

- Figure 6: shows the binding characteristics of the clone K8V_{K}/dummy V_{H} analysed using soluble scFv ELISA. Production of the soluble scFv fragments was induced by IPTG as described by Harrison et al, Methods Enzymol. 1996;267:83-109 and the supernatant containing scFv assayed directly. Soluble scFv ELISA is performed as described in example 1 and the bound scFvs were detected with Protein L-HRP. The ELISA results revealed that this clone was still able to bind β-gal, whereas binding BSA was abolished.
- Figure 7: shows the binding of dual specific scFv antibodies directed against APS and β-gal and a dual specific scFv antibody directed against BCL10 protein and β-gal to their respective antigen.

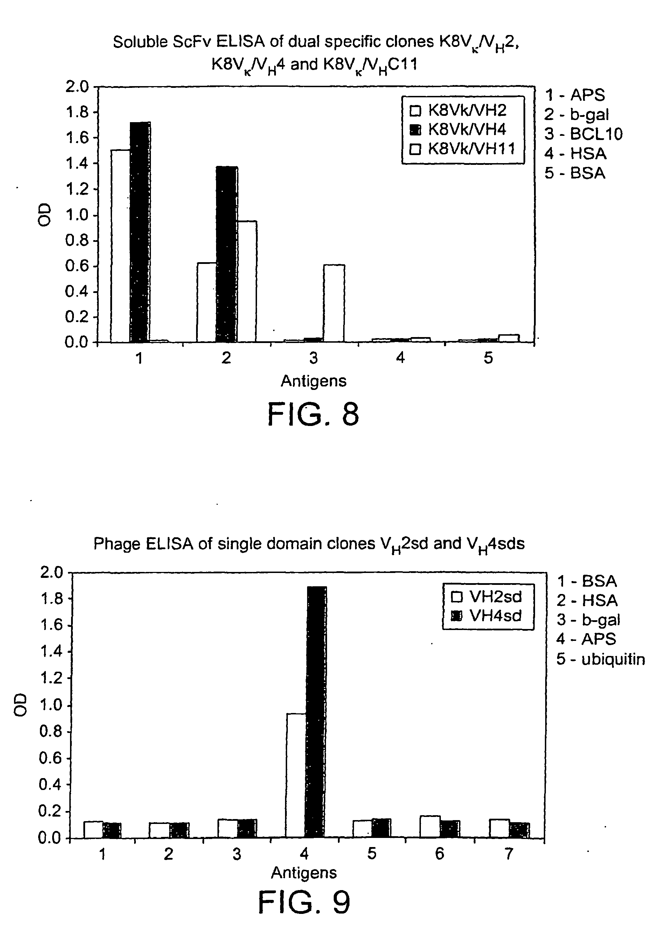

- Figure 8: shows the binding characteristics of K8V_{K}/V_{H}2/K8V_{K}/V_{H}4 and K8V_{K}/V_{H}C11 using a soluble scFv ELISA as described herein. All clones were dual specific without any cross-reactivity with other proteins.
- Figure 9: shows the binding characteristics of produced clones V_{H}2sd and V_{H}4sd tested by monoclonal phage ELISA. Phage particles were produced as described by Harrison *et al* in 1996. 96-well ELISA plates were coated with 100µg/ml of APS, BSA, HSA, β-gal, ubiquitin, α-amylase and myosin at 10µg/ml concentration in PBS overnight at 4°C. A standard ELISA protocol was followed using detection of bound phage with anti-M13-HRP conjugate. ELISA results demonstrated that VH single domains specifically recognised APS when displayed on the surface of the filamentous bacteriophage.

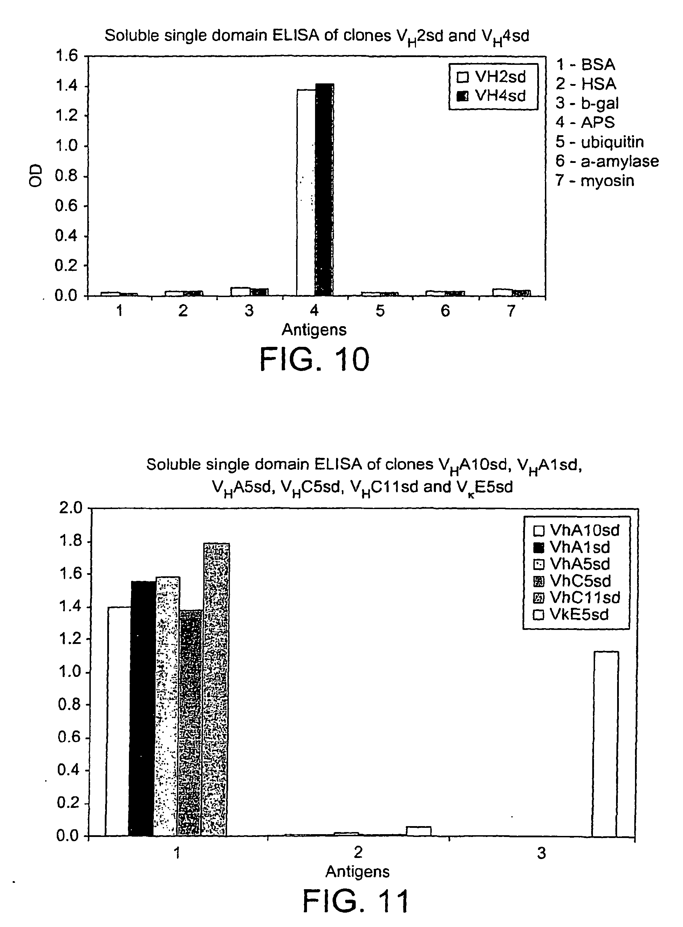

- Figure 10: shows the ELISA of soluble V_{H}2sd and V_{H}4sd. The same results are obtained as with the phage ELISA shown in figure 9, indicating that these single domains are also able to recognise APS or soluble fragments.
- Figure 11: shows the selection of single V_{H} domain antibodies directed against APS and single V_{K} domain antibodies directed against β-gal from a repertoire of single antibody domains. Soluble single domain ELISA was performed as soluble scFv ELISA described in example 1 and bound V_{K} and V_{H} single domains were detected with Protein L-HRP and Protein A-HRP respectively. Five VH single domains V_{H}A10sd, V_{H}A1sd, V_{H}A5sd, V_{H}C5sd and V_{H}C11sd selected from library 5 were found to bind APS and one V_{K} single domain V_{K}E5SD selected from library 6 was found to bind β -gal. None of the clones cross-reacted with BSA.

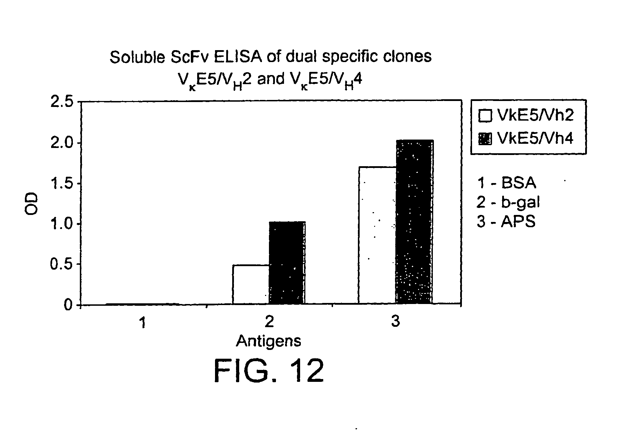

- Figure 12: shows the characterisation of dual specific scFv antibodies V_{K}E5/V_{H}2 and V_{K}E5/V_{H}4 directed against APS and β-gal. Soluble scFv ELISA was performed as described in example 1 and the bound scFvs were detected with Protein L-HRP. Both V_{K}E5/V_{H}2 and V_{K}E5/V_{H}4 clones were found to be dual specific. No cross reactivity with BSA was detected.

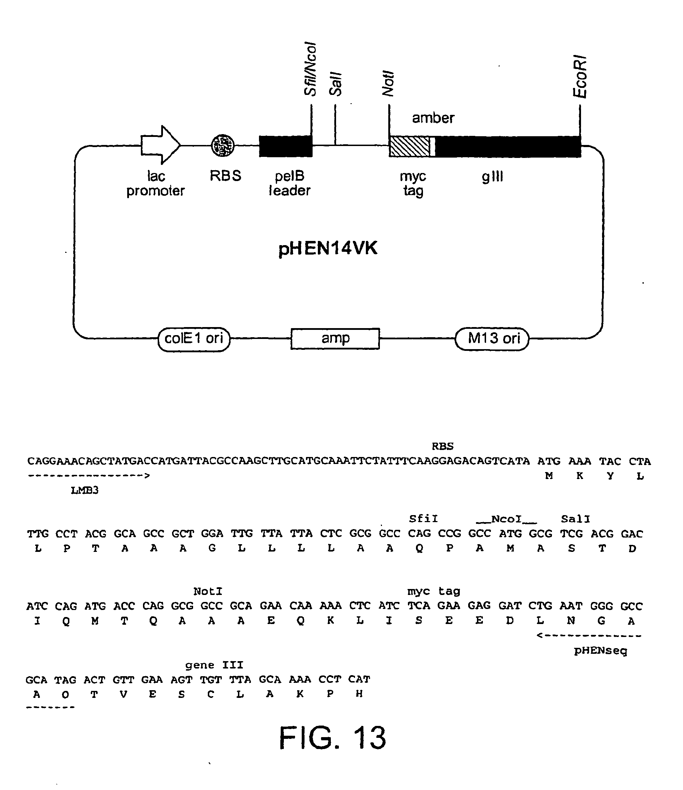

- Figure 13: shows the construction of V_{K} vector and V_{K}G3 vector. V_{K}G was pc amplified from an individual clone, A4 selected from a Fab library using BK BACKNOT as a 5' back primer and CKSACFORFL as a 3' (forward) primer. 30 cycles of PCR amplification was performed except that P*fu* polymerase was used in enzyme. PCR product was digested with Not*I*/*EcoRI* and ligated into a Not*IEcoRI* digested vector pHEN14V_{K} to create a C_{K} vector.

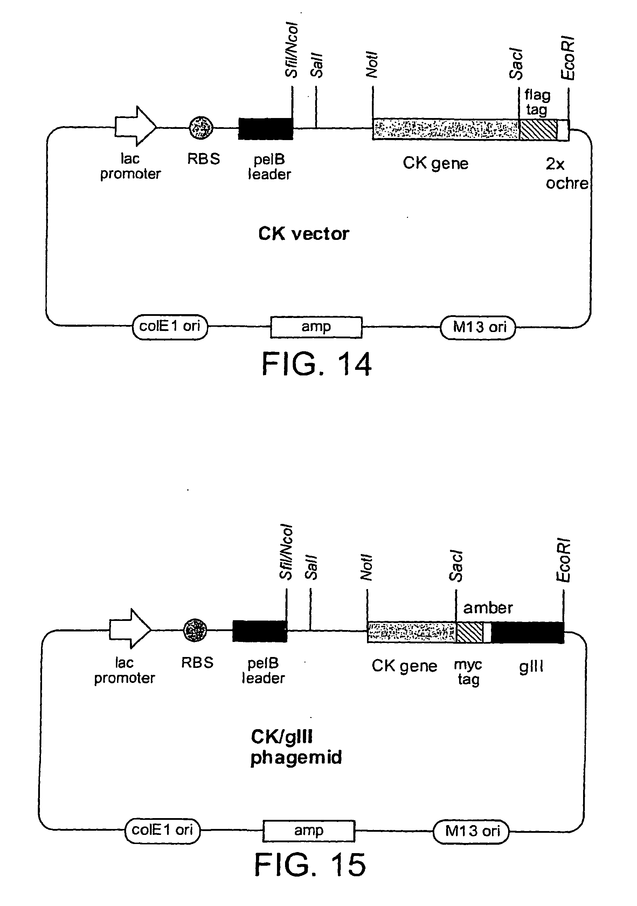

- Figure 14: shows the C_{K} vector referred to in figure 13.
- Figure 15: shows a Ck/gIII phagemid. Gene III was PCR amplified from a pIT2 vector using G3BACKSAC as a 5' (back) primer and LMB2 as a 3' (forward) primer. 30 cycles of PCR amplification were performed as described herein. PCR product was digested with SAC*I*/EcoR*I* and ligated into a Sac*I*/EcoR*I* digested C_{K} vector.

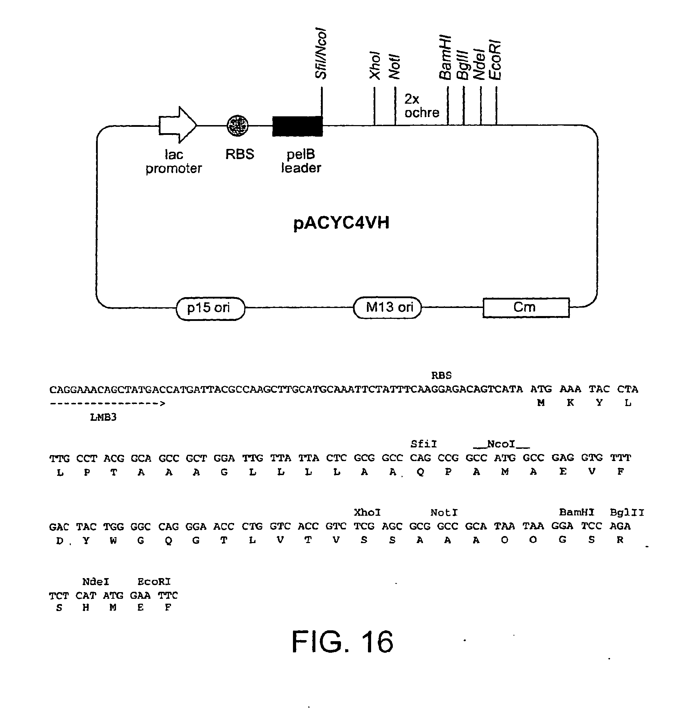

- Figure 16: shows a C_{H} vector. C_{H} gene was PCR amplified from an individual clone A4 selected from a Fab library using CHBACKNOT as a 5' (back) primer and CHSACFOR as a 3' (forward) primer. 30 cycles of PCR amplification were performed as described herein. PCR product was digested with a Not*I*/Bg1*II* and ligated into a Not*I*/Bg1*II* digested vector PACYC4V_{H} to create a C_{H} vector.

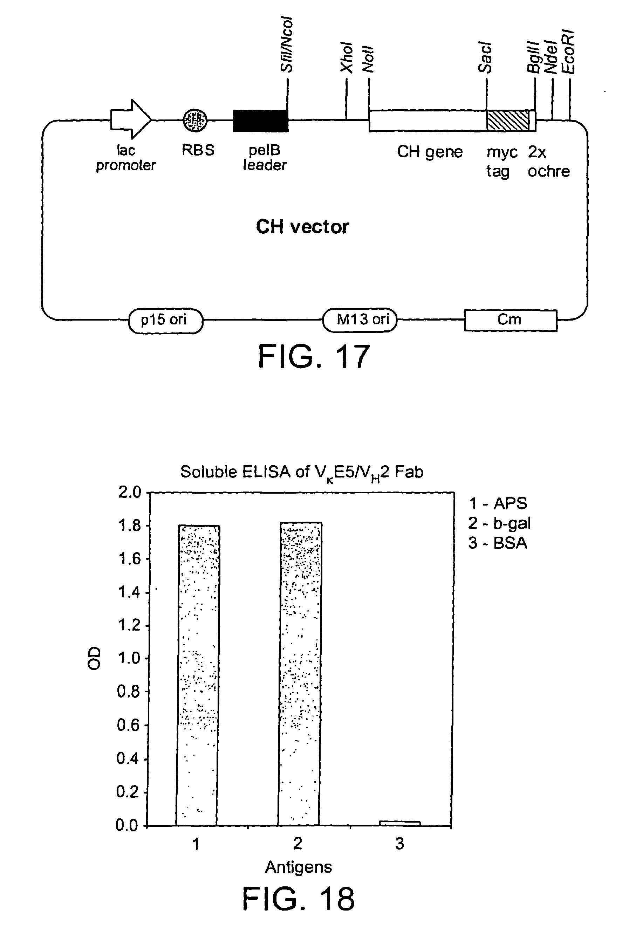

- Figure 17: shows the C_{H} vector referred to in Figure 16.
- Figure 18: shows an ELISA of V_{κ} E5/V_{H}2 Fab.

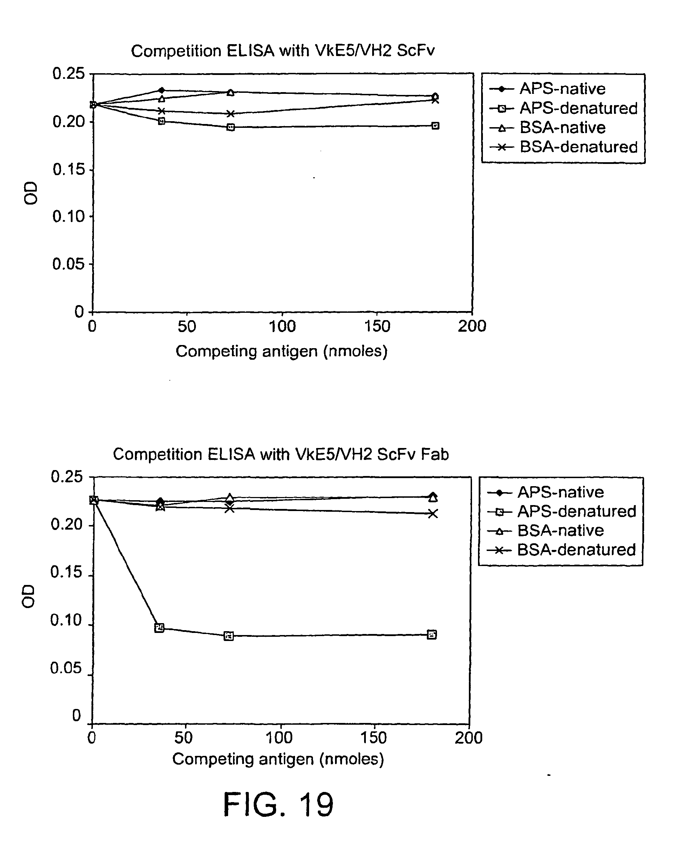

- Figure 19: shows competition ELISAs with V_{κ}E5/V_{H}2 scFv and V_{κ}E5/V_{H}2 Fab.

### Detailed Description of the invenion

### Definitions

**Complementary** Two immunoglobulin domains are "complementary" where they belong to families of structures which form cognate pairs or groups or are derived from such families and retain this feature. For example, a V_{H} domain and a V_{L} domain of an antibody are complementary; two V_{H} domains are not complementary, and two V_{L} domains are not complementary. Complementary domains may be found in other members of the immunoglobulin superfamily, such as the V_{α} and V_{β} (or γ and δ) domains of the T-cell receptor. In the context of the present invention, complementary domains do not bind a target molecule co-operatively, but act independently on different target epitopes which may be on the same or different molecules.

**Immunoglobulin** This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular *interactions in vivo,* including widespread roles in the immune system (for example, antibodies, T-cell receptor molecules and the like), involvement in cell adhesion (for example the ICAM molecules) and intracellular signalling (for example, receptor molecules, such as the PDGF receptor). The present invention is applicable to all immunoglobulin superfamily molecules which possess complementary domains. Preferably, the present invention relates to antibodies.

**Combining** Complementary variable domains according to the invention are combined to form a group of complementary domains; for example, V_{L} domains are combined with V_{H} domains. Domains may be combined in a number of ways, involving linkage of the domains by covalent or non-covalent means.

**Domain** A domain is a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain. By single antibody variable domain we mean a folded polypeptide domain comprising sequences characteristic of antibody variable domains. It therefore includes complete antibody variable domains and modified variable domains, for example in which one or more loops have been replaced by sequences which are not characteristic of antibody variable domains, or antibody variable domains which have been truncated or comprise N- or C-terminal extensions, as well as folded fragments of variable domains which retain at least in part the binding activity and specificity of the full-length domain.

**Repertoire** A collection of diverse variants, for example polypeptide variants which differ in their primary sequence. A library used in the present invention will encompass a repertoire of polypeptides comprising at least 1000 members.

**Library** The term library refers to a mixture of heterogeneous polypeptides or nucleic acids. The library is composed of members, which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

**Antibody** An antibody (for example IgG, IgM, IgA, IgD or IgE) or fragment (such as a Fab , F(Ab')₂, Fv, disulphide linked Fv, scFv, disulphide-linked scFv, diabody) whether derived from any species naturally producing an antibody, or created by recombinant DNA technology; whether isolated from serum, B-cells, hybridomas, transfectomas, yeast or bacteria).

**Dual-specific ligand** A ligand comprising a first immunoglobulin single variable domain and a second immunoglobulin single variable domain as herein defined, wherein the variable regions are capable of binding to two different antigens or two epitopes on the same antigen which are not normally bound by a monospecific immunoglobulin. For example, the two epitopes may be on the same hapten, but are not the same epitope or sufficiently adjacent to be bound by a monospecific ligand. The dual specific ligands according to the invention are composed of mutually complementary variable domain pairs which have different specificities, and do not contain mutually complementary variable domain pairs which have the same specificity.

**Antigen** A ligand that binds to a small fraction of the members of a repertoire according to the present invention. It may be a polypeptide, protein, nucleic acid or other molecule. Generally, the dual specific ligands according to the invention are selected for target specificity against a particular antigen. In the case of conventional antibodies and fragments thereof, the antibody binding site defined by the variable loops (L1, L2, L3 and H1, H2, H3) is capable of binding to the antigen.

**Epitope** A unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation.

**Specific generic ligand** A ligand that binds to all members of a repertoire. Generally, not bound through the antigen binding site as defined above. Examples include protein A and protein L.

**Selecting** Derived by screening, or derived by a Darwinian selection process, in which binding interactions are made between a domain and the antigen or epitope or between an antibody and an antigen or epitope. Thus a first variable domain may be selected for binding to an antigen or epitope in the presence or in the absence of a complementary variable domain.

**Universal framework** A single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917, The invention provides for the use of a single framework, or a set of such frameworks, which has been found to permit the derivation of virtually any binding specificity though variation in the hypervariable regions alone.

### Detailed description of the invention

Unless defined otherwise, all technical and scientific terms used herein have the same meaning as commonly understood by one of ordinary skill in the art (e.g., in cell culture, molecular genetics, nucleic acid chemistry, hybridisation techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. and chemical methods.

Dual specific ligands according to the invention may be prepared according to previously established techniques, used in the field of antibody engineering, for the preparation of scFv, "phage" antibodies and other engineered antibody molecules. Techniques for the preparation of antibodies, and in particular bispecific antibodies, are for example described in the following reviews and the references cited therein: Winter & Milstein, (1991) Nature 349:293-299; Plueckthun (1992) Immunological Reviews 130:151-188; Wright et al., (1992) Crti. Rev. Immunol.12:125-168; Holliger, P. & Winter, G. (1993) Curr. Op. Biotechn. 4, 446-449; Carter, et al. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126; Fearon, D. (1997) Nature Biotechnol. 15, 618-619; Plückthun, A. & Pack, P. (1997) Immunotechnology 3, 83-105; Carter, P. & Merchant, A.M. (1997) Curr. Opin. Biotechnol. 8, 449-454; Holliger, P. & Winter, G. (1997) Cancer Immunol. Immunother. 45,128-130.

The disclosure provides for the selection of complementary variable domains against two different antigens or epitopes, and subsequent combination of the variable domains.

The techniques employed for selection of the variable domains employ libraries and selection procedures which are known in the art. Natural libraries (Marks et al. (1991) J. Mol. Biol., 222: 581; Vaughan et al. (1996) Nature Biotech., 14: 309) which use rearranged V genes harvested from human B cells are well known to those skilled in the art. Synthetic libraries (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97) are prepared by cloning immunoglobulin V genes, usually using PCR. Errors in the PCR process can lead to a high degree of randomisation. V_{H} and/or V_{L} libraries may be selected against target antigens or epitopes separately, in which case single domain binding is directly selected for, or together.

A preferred method for making a dual specific ligand according to the present invention comprises using a selection system in which a repertoire of variable domains is selected for binding to a first antigen or epitope and a repertoire of variable domains is selected for binding to a second antigen or epitope. The selected variable first and second variable domains are then combined and the dual-specific selected for binding to both first and second antigen or epitope.

### A. Library vector systems

A variety of selection systems are known in the art which are suitable for use in the present invention. Examples of such systems are described below.

Bacteriophage lambda expression systems may be screened directly as bacteriophage plaques or as colonies of lysogens, both as previously described (Huse et al. (1989) Science, 246: 1275; Caton and Koprowski (1990) Proc. Natl. Acad. Sci. U.S.A., 87*;* Mullinax et al. (1990) Proc. Natl. Acad. Sci. U.S.A., 87: 8095; Persson et al. (1991) Proc. Natl. Acad. Sci. U.S.A., 88: 2432) and are of use in the invention. Whilst such expression systems can be used to screening up to 10⁶ different members of a library, they are not really suited to screening of larger numbers (greater than 10⁶ members).

Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences that encoding them) for the *in vitro* selection and amplification of specific antibody fragments that bind a target antigen (McCafferty et al., WO 92/01047). The nucleotide sequences encoding the V_{H} and V_{L} regions are linked to gene fragments which encode leader signals that direct them to the periplasmic space of *E. coli* and as a result the resultant antibody fragments are displayed on the surface of the bacteriophage, typically as fusions to bacteriophage coat proteins (e.g., pIII or pVIII). Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks *et al.* (1991) supra; Barbas *et al.* (1992) supra; Hawkins and Winter (1992) J. Immunol., 22: 867; Marks et al., 1992, J. Biol. Chem., 267: 16007; Lerner et al. (1992) Science, 258: 1313.).

One particularly advantageous approach has been the use of scFv phage-libraries (Huston et al., 1988, Proc. Natl. Acad. Sci U.S.A., 85: 5879-5883; Chaudhary et al. (1990) Proc. Natl. Acad. Sci U.S.A., 87: 1066-1070; McCafferty *et al.* (1990) supra; Clackson et al. (1991) Nature, 352: 624; Marks et al. (1991) J. Mol. Biol., 222: 581; Chiswell et al. (1992) Trends Biotech., 10: 80; Marks et al. (1992) J. Biol. Chem., 267). Various embodiments of scFv libraries displayed on bacteriophage coat proteins have been described. Refinements of phage display approaches are also known, for example as described in WO96/06213 and WO92/01047 (Medical Research Council *et al.*) and WO97/08320 (Morphosys).

Other systems for generating libraries of polypeptides involve the use of cell-free enzymatic machinery for the *in vitro* synthesis of the library members. In one method, RNA molecules are selected by alternate rounds of selection against a target ligand and PCR amplification (Tuerk and Gold (1990) Science, 249: 505; Ellington and Szostak (1990) Nature, 346: 818). A similar technique may be used to identify DNA sequences which bind a predetermined human transcription factor (Thiesen and Bach (1990) Nucleic Acids Res., 18: 3203; Beaudry and Joyce (1992) Science, 257: 635; WO92/05258 and WO92/14843). In a similar way*, in vitro* translation can be used to synthesise polypeptides as a method for generating large libraries. These methods which generally comprise stabilised polysome complexes, are described further in WO88/08453, WO90/05785, WO90/07003, WO91/02076, WO91/05058, and WO92/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671, WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### B. Library Construction.

Libraries intended for selection, may be constructed using techniques known in the art, for example as set forth above, or may be purchased from commercial sources. Libraries which are useful in the present invention are described, for example, in WO99/20749. Once a vector system is chosen and one or more nucleic acid sequences encoding polypeptides of interest are cloned into the library vector, one may generate diversity within the cloned molecules by undertaking mutagenesis prior to expression; alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) Methods Enzymol., 155: 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter et al. (1994) Ann. Rev. Immunology 12,433-55, and references cited therein.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenized, mismatch is required, at least in the first round of synthesis. The ability to optimise the stringency of primer annealing conditions is well within the knowledge of one of moderate skill in the art. An annealing temperature of between 30 C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### C. Combining complementary single domains

Domains according to the invention, once selected, may be combined by a variety of methods known in the art, including covalent and non-covalent methods.

Preferred methods include the use of polypeptide linkers, as described, for example, in connection with scFv molecules (Bird et al., (1988) Science 242:423-426). Linkers are preferably flexible, allowing the two single domains to interact. The linkers used in diabodies, which are less flexible, may also be employed (Holliger et al., (1993) PNAS (USA) 90:6444-6448).

Complementary variable domains may be combined using methods other than linkers. For example, the use of disulphide bridges, provided through naturally-occurring or engineered cysteine residues, may be exploited to stabilise V_{H}-V_{L} dimers (Reiter et al., (1994) Protein Eng. 7:697-704) or by remodelling the interface between the variable domains to improve the "fit" and thus the stability of interaction (Ridgeway et al., (1996) Protein Eng. 7:617-621; Zhu et al., (1997) Protein Science 6:781-788).

Other techniques for joining or stabilising variable domains of immunoglobulins, and in particular antibody V_{H} and V_{L} domains, may be employed as appropriate.

It is envisaged that dual specific ligands may exist in "open" or "closed" conformations in solution. An "open" conformation is a conformation in which each of the immunoglobulin domains is present in a form unassociated with other domains; in other words, each domain is present as a single domain in solution (albeit combined, e.g. *via* a linker, with the other domain). The "closed" configuration is that in which the two domains (for example V_{H} and V_{L}) are present in associated form, such as that of an associated V_{H}-V_{L} pair which forms an antibody binding site. For example, scFv may be in a closed or open conformation, depending on the arrangement of the linker used to link the V_{H} and V_{L} domains. If this is sufficiently flexible to allow the domains to associate, or rigidly holds them in the associated position, it is likely that the domains will adopt a closed conformation. A short or rigid linker may however be used to keep V_{H} and V_{L} domains apart, and prevent a closed conformation from forming.

Fab fragments and whole antibodies will exist primarily in the closed conformation, although it will be appreciated that open and closed dual specific ligands are likely to exist in a variety of equilibria under different circumstances. Binding of the ligand to a target is likely to shift the balance of the equilibrium towards the open configuration. Thus, the ligands according to the invention can exist in two conformations in solution, one of which (the open form) can bind two antigens or epitopes independently, whilst the alternative conformation (the closed form) can only bind one antigen or epitope; antigens or epitopes thus compete for binding to the ligand in this conformation.

Although the open form of the dual specific ligand may thus exist in equilibrium with the closed form solution, it is envisaged that the equilibrium will favour the closed form; moreover, the open form can be sequestered by target binding into a closed conformation.

Preferably, therefore, the dual specific ligand is present in an equilibrium between two (open and closed) conformations.

Dual specific ligands may be modified in order to favour an open or closed conformation. For example, stabilisation of V_{H}-V_{L} interactions with disulphide bonds stabilises the closed conformation. Moreover, linkers used to join the domains may be constructed such that the open from is favoured; for example, the linkers may sterically hinder the association of the domains, such as by incorporation of large amino acid residues in opportune locations, or the designing of a suitable rigid structure which will keep the domains physically spaced apart.

### D. Characterisation of the dual-specific ligand.

The binding of the dual-specific ligand to its specific antigens or epitopes can be tested by methods which will be familiar to those skilled in the art and include ELISA. In a preferred embodiment of the invention binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12,433-55 and references cited therein.

The diversity of the selected phage monoclonal antibodies may also be assessed by gel electrophoresis of PCR products (Marks et al. 1991, *supra;* Nissim et al. 1994 *supra*)*,* probing (Tomlinson et al., 1992) J. Mol. Biol. 227, 776) or by sequencing of the vector DNA.

### E. Structure of 'Dual-specific ligands'.

As described above, an antibody is herein defined as an antibody (for example IgG, IgM, IgA, IgA, IgE) or fragment (Fab, Fv, disulphide linked Fv, scFv, diabody) which comprises at least one heavy and a light chain variable domain which are complementary to one another and thus can associate with one another to form a VH/VL pair. It may be derived from any species naturally producing an antibody, or created by recombinant DNA technology; whether isolated from serum, B-cells, hybridomas, transfectomas, yeast or bacteria).

In a preferred embodiment the dual-specific ligand comprises at least one single heavy chain variable domain of an antibody and one single light chain variable domain of an antibody such that the two regions are capable of associating to form a complementary VH/VL pair.

The first and the second variable domains of such a ligand may be on the same polypeptide chain. Alternatively they may be on separate polypeptide chains. In the case that they are on the same polypeptide chain they may be linked by a flexible linker, which is preferentially a peptide sequence, as described above.

The first and second variable domains may be covalently or non-covalently associated. In the case that they are covalently associated, the covalent bonds may be disulphide bonds.

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair.

In a further aspect, the present invention provides nucleic acid encoding at least a 'dual-specific ligand' as defined in claim 1.

One skilled in the art will appreciate that both antigens or epitopes may bind simultaneously to the same antibody molecule. Alternatively, they may compete for binding to the same antibody molecule. For example, where both epitopes are bound simultaneously, both V_{H} and V_{L} domains of a dual specific ligand are able to independently bind their target epitopes. Where the domains compete, the V_{H} is capable of binding its target, but not at the same time as the V_{L} binds its cognate target; or the V_{L} is capable of binding its target, but not at the same time as the V_{H} binds its cognate target.

The variable regions may be derived from antibodies directed against target antigens or epitopes. Alternatively they may be derived from a repertoire of single antibody domains such as those expressed on the surface of filamentous bacteriophage. Selection may be performed as described below.

In general, the nucleic acid molecules and vector constructs required for the performance of the present invention may be constructed and manipulated as set forth in standard laboratory manuals, such as Sambrook et al. (1989) Molecular Cloning: A Laboratory Manual, Cold Spring Harbor, USA.

The manipulation of nucleic acids in the present invention is typically carried out in recombinant vectors.

Thus in a further aspect, the present invention provides a vector comprising nucleic acid encoding at least a 'dual-specific ligand' as defined in claim 1.

As used herein, vector refers to a discrete element that is used to introduce heterologous DNA into cells for the expression and/or replication thereof. Methods by which to select or construct and, subsequently, use such vectors are well known to one of moderate skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length A suitable host cell is transformed with the vector after *in vitro* cloning manipulations. Each vector contains various functional components, which generally include a cloning (or "polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a polypeptide repertoire member according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Since the replication of vectors according to the present invention is most conveniently performed in *E*. *coli,* an *E*. *coli*-selectable marker, for example, the β-lactamase gene that confers resistance to the antibiotic ampicillin, is of use. These can be obtained from *E*. *coli* plasmids, such as pBR322 or a pUC plasmid such as pUC18 or pUC19.

Expression vectors usually contain a promoter that is recognised by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence.

The preferred vectors are expression vectors that enables the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used. The preferred vectors are phagemid vectors which have an *E*. *coli.* origin of replication (for double stranded replication) and also a phage origin of replication (for production of single-stranded DNA). The manipulation and expression of such vectors is well known in the art (Hoogenboom and Winter (1992) supra; Nissim *et al.* (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non-suppressor strains of *E. coli* and with the addition of glucose, iso-propyl thio-β-D-galactoside (IPTG) or a helper phage, such as VCS M13, the vector is able to replicate as a plasmid with no expression, produce large quantities of the polypeptide library member only or produce phage, some of which contain at least one copy of the polypeptide-pIII fusion on their surface.

Construction of vectors according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, *in situ* hybridisation, immunocytochemistry or sequence analysis of nucleic acid or protein molecules. Those skilled in the art will readily envisage how these methods may be modified, if desired.

### F: Scaffolds for use in Constructing Dual Specific Ligands

### i. Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol.,196: 901; Chothia et al. (1989) Nature, 342: 877).

Analysis of loop lengths and key residues has therefore enabled prediction of the main-chain conformations of H1, H2, L1, L2 and L3 found in the majority of human antibodies (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1).

The dual specific ligands of the present invention are advantageously assembled from libraries of domains, such as libraries of V_{H} domains and libraries of V_{L} domains. Moreover, the dual specific ligands of the invention may themselves be provided in the form of libraries. In one aspect of the present invention, libraries of dual specific ligands and/or domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main-chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human V_{κ} domain, the L1 loop can adopt one of four canonical structures, the L2 loop has a single canonical structure and that 90% of human V_{κ} domains adopt one of four or five canonical structures for the L3 loop (Tomlinson *et al.* (1995) supra); thus, in the V_{κ} domain alone, different canonical structures can combine to create a range of different main-chain conformations. Given that the V_{λ} domain encodes a different range of canonical structures for the L1, L2 and L3 loops and that V_{κ} and V_{λ} domains can pair with any V_{H} domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a preferred aspect, the dual-specific ligands of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing dual specific ligands or libraries thereof the incidence of the different main-chain conformations for each of the six antigen binding loops may be considered separately. For H1, H2, L1, L2 and L3, a given conformation that is adopted by between 20% and 100% of the antigen binding loops of naturally occurring molecules is chosen. Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of V_{κ} (39%), L2 - CS 1 (100%), L3 - CS 1 of V_{κ} (36%) (calculation assumes a κ:λ ratio of 70:30, Hood et al. (1967) Cold Spring Harbor Symp. Quant. Biol-., 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the V_{H} segment 3-23 (DP-47), the J_{H} segment JH4b, the V_{κ} segment O2/O12 (DPK9) and the J_{κ} segment J_{κ}1. V_{H} segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

### ii. Diversification of the canonical sequence

Having selected several known main-chain conformations or, preferably a single known main-chain conformation, dual specific ligands according to the invention or libraries for use in the invention can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or are preferably selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error-prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J. Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri et al. (1996) Nature Med., 2: 100; Riechmann et al. (1995) Bio/Technology, 13: 475; Morphosys, WO97/08320, supra).

Since loop randomisation has the potential to create approximately more than 10¹⁵ structures for H3 alone and a similarly large number of variants for the other five loops, it is not feasible using current transformation technology or even by using cell free systems to produce a library representing all possible combinations. For example, in one of the largest libraries constructed to date, 6 × 10¹⁰ different antibodies, which is only a fraction of the potential diversity for a library of this design, were generated (Griffiths *et al.* (1994) supra).

In a preferred embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main-chain conformation.

### Diversification of the canonical sequence as it applies to antibody domains

In the case of antibody dual-specific ligands, the binding site for the target is most often the antigen binding site. Thus, in a highly preferred aspect, the invention provides libraries of or for the assembly of antibody dual-specific ligands in which only those residues in the antigen binding site are varied. These residues are extremely diverse in the human antibody repertoire and are known to make contacts in high-resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat *et al.* (1991, supra), some seven residues compared to the two diversified in the library for use in to the invention. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarity has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

The invention provides two different naive repertoires of binding domains for the construction of dual specific ligands, or a naive library of dual specific ligands, in which some or all of the residues in the antigen binding site are varied. The "primary" library mimics the natural primary repertoire, with diversity restricted to residues at the centre of the antigen binding site that are diverse in the germline V gene segments (germline diversity) or diversified during the recombination process (junctional diversity). Those residues which are diversified include, but are not limited to, H50, H52, H52a, H53, H55, H56, H58, H95, H96, H97, H98, L50, L53, L91, L92, L93, L94 and L96. In the "somatic" library, diversity is restricted to residues that are diversified during the recombination process (junctional diversity) or are highly somatically mutated). Those residues which are diversified include, but are not limited to: H31, H33, H35, H95, H96, H97, H98, L30, L31, L32, L34 and L96. All the residues listed above as suitable for diversification in these libraries are known to make contacts in one or more antibody-antigen complexes. Since in both libraries, not all of the residues in the antigen binding site are varied, additional diversity is incorporated during selection by varying the remaining residues, if it is desired to do so. It shall be apparent to one skilled in the art that any subset of any of these residues (or additional residues which comprise the antigen binding site) can be used for the initial and/or subsequent diversification of the antigen binding site.

In the construction of libraries for use in the invention, diversification of chosen positions is typically achieved at the nucleic acid level, by altering the coding sequence which specifies the sequence of the polypeptide such that a number of possible amino acids (all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA.

A feature of side-chain diversity in the antigen binding site of human antibodies is a pronounced bias which favours certain amino acid residues. If the amino acid composition of the ten most diverse positions in each of the V_{H}, V_{κ} and V_{λ} regions are summed, more than 76% of the side-chain diversity comes from only seven different residues, these being, serine (24%), tyrosine (14%), asparagine (11%), glycine (9%), alanine (7%), aspartate (6%) and threonine (6%). This bias towards hydrophilic residues and small residues which can provide main-chain flexibility probably reflects the evolution of surfaces which are predisposed to binding a wide range of antigens or epitopes and may help to explain the required promiscuity of antibodies in the primary repertoire.

Since it is preferable to mimic this distribution of amino acids, the distribution of amino acids at the positions to be varied preferably mimics that seen in the antigen binding site of antibodies. Such bias in the substitution of amino acids that permits selection of certain polypeptides (not just antibody polypeptides) against a range of target antigens is easily applied to any polypeptide repertoire. There are various methods for biasing the amino acid distribution at the position to be varied (including the use of tri-nucleotide mutagenesis, see WO97/08320), of which the preferred method, due to ease of synthesis, is the use of conventional degenerate codons. By comparing the amino acid profile encoded by all combinations of degenerate codons (with single, double, triple and quadruple degeneracy in equal ratios at each position) with the natural amino acid use it is possible to calculate the most representative codon. The codons (AGT)(AGC)T, (AGT)(AGC)C and (AGT)(AGC)(CT) - that is, DVT, DVC and DVY, respectively using IUPAC nomenclature - are those closest to the desired amino acid profile: they encode 22% serine and 11% tyrosine, asparagine, glycine, alanine, aspartate, threonine and cysteine. Preferably, therefore, libraries are constructed using either the DVT, DVC or DVY codon at each of the diversified positions.

### G: Use of dual-specific ligands according to the invention

Dual-specific ligands selected according to the method of the present disclosure may be employed in in vivo therapeutic and prophylactic applications, in vitro and in vivo diagnostic applications, in vitro assay and reagent applications, and the like. For example antibody molecules may be used in antibody based assay techniques, such as ELISA techniques, according to methods known to those skilled in the art.

As alluded to above, the molecules selected according to the invention are of use in diagnostic, prophylactic and therapeutic procedures. Dual specific antibodies selected according to the disclosure are of use diagnostically in Western analysis and *in situ* protein detection by standard immunohistochemical procedures; for use in these applications, the antibodies of a selected repertoire may be labelled in accordance with techniques known to the art. In addition, such antibody polypeptides may be used preparatively in affinity chromatography procedures, when complexed to a chromatographic support, such as a resin. All such techniques are well known to one of skill in the art.

Diagnostic uses of the dual specific ligands according to the invention include homogenous assays for analytes which exploit the ability of dual specific ligands to bind two targets in competition, such that two targets cannot bind simultaneously (a closed conformation), or alternatively their ability to bind two targets simultaneously (an open conformation).

A true homogenous immunoassay format has been avidly sought by manufacturers of diagnostics and research assay systems used in drug discovery and development. The main diagnostics markets include human testing in hospitals, doctor's offices and clinics, commercial reference laboratories, blood banks, and the home, non-human diagnostics (for example food testing, water testing, environmental testing, bio-defence, and veterinary testing), and finally research (including drug development; basic research and academic research).

At present all these markets utilise immunoassay systems that are built around chemiluminescent, ELISA, fluorescence or in rare cases radio-immunoassay technologies. Each of these assay formats requires a separation step (separating bound from un-bound reagents). In some cases, several separation steps are required. Adding these additional steps adds reagents and automation, takes time, and affects the ultimate outcome of the assays. In human diagnostics, the separation step may be automated, which masks the problem, but does not remove it. The robotics, additional reagents, additional incubation times, and the like add considerable cost and complexity. In drug development, such as high throughput screening, where literally millions of samples are tested at once, with very low levels of test molecule, adding additional separation steps can eliminate the ability to perform a screen. However, avoiding the separation creates too much noise in the read out. Thus, there is a need for a true homogenous format that provides sensitivities at the range obtainable from present assay formats. Advantageously, an assay possesses fully quantitative read-outs with high sensitivity and a large dynamic range. Sensitivity is an important requirement, as is reducing the amount of sample required. Both of these features are features that a homogenous system offers. This is very important in point of care testing, and in drug development where samples are precious. Heterogenous systems, as currently available in the art, require large quantities of sample and expensive reagents

Applications for homogenous assays include cancer testing, where the biggest assay is that for Prostate Specific Antigen, used in screening men for prostate cancer. Other applications include fertility testing, which provides a series of tests for women attempting to conceive including beta-hcg for pregnancy. Tests for infectious diseases, including hepatitis, HIV, rubella, and other viruses and microorganisms and sexually transmitted diseases. Tests are used by blood banks, especially tests for HIV, hepatitis A, B, C, non A non B. Therapeutic drug monitoring tests include monitoring levels of prescribed drugs in patients for efficacy and to avoid toxicity, for example digoxin for arrhythmia, and phenobarbital levels in psychotic cases; theophylline for asthma. Diagnostic tests are moreover useful in abused drug testing, such as testing for cocaine, marijuana and the like. Metabolic tests are used for measuring thyroid function, anaemia and other physiological disorders and functions.

The homogenous immunoassay format is moreover useful in the manufacture of standard clinical chemistry assays. The inclusion of immunoassays and chemistry assays on the same instrument is highly advantageous in diagnostic testing. Suitable chemical assays include tests for glucose, cholesterol, potassium, and the like.

A further major application for homogenous immunoassays is drug discovery and development: High throughput screening includes testing combinatorial chemistry libraries versus targets in ultra high volume. Signal is detected, and positive groups then split into smaller groups, and eventually tested in cells and then animals. Homogenous assays may be used in all these types of test. In drug development, especially animal studies and clinical trials heavy use of immunoassays is made. Homogenous assays greatly accelerate and simplify these procedures. Other Applications include food and beverage testing: testing meat and other foods for E. coli, salmonella, etc; water testing, including testing at water plants for all types of contaminants including E. coli; and veterinary testing.

In a broad embodiment, the invention provides a binding assay comprising a detectable agent which is bound to a dual specific ligand according to the invention, and whose detectable properties are altered by the binding of an analyte to said dual specific ligand. Such an assay may be configured in several different ways, each exploiting the above properties of dual specific ligands.

Where the dual specific ligand is in a closed conformation, the assay relies on the direct or indirect displacement of an agent by the analyte, resulting in a change in the detectable properties of the agent. For example, where the agent is an enzyme which is capable of catalysing a reaction which has a detectable end-point, said enzyme can be bound by the ligand such as to obstruct its active site, thereby inactivating the enzyme. The analyte, which is also bound by the dual specific ligand, displaces the enzyme, rendering it active through freeing of the active site. The enzyme is then able to react with a substrate, to give rise to a detectable event. In an alternative embodiment, the ligand may bind the enzyme outside of the active site, influencing the conformation of the enzyme and thus altering its activity. For example, the structure of the active site may be constrained by the binding of the ligand, or the binding of cofactors necessary for activity may be prevented.

The physical implementation of the assay may take any form known in the art. For example, the dual specific ligand/enzyme complex may be provided on a test strip; the substrate may be provided in a different region of the test strip, and a solvent containing the analyte allowed to migrate through the ligand/enzyme complex, displacing the enzyme, and carrying it to the substrate region to produce a signal. Alternatively, the ligand/enzyme complex may be provided on a test stick or other solid phase, and dipped into an analyte/substrate solution, releasing enzyme into the solution in response to the presence of analyte.

Since each molecule of analyte potentially releases one enzyme molecule, the assay is quantitative, with the strength of the signal generated in a given time being dependent on the concentration of analyte in the solution.

Further configurations using the analyte in a closed conformation are possible. For example, the dual specific ligand may be configured to bind an enzyme in an allosteric site, thereby activating the enzyme. In such an embodiment, the enzyme is active in the absence of analyte. Addition of the analyte displaces the enzyme and removes allosteric activation, thus inactivating the enzyme.

In the context of the above embodiments which employ enzyme activity as a measure of the analyte concentration, activation or inactivation of the enzyme refers to an increase or decrease in the activity of the enzyme, measured as the ability of the enzyme to catalyse a signal-generating reaction. For example, the enzyme may catalyse the conversion of an undetectable substrate to a detectable form thereof. For example, horseradish peroxidase is widely used in the art together with chromogenic or chemiluminescent substrates, which are available commercially. The level of increase or decrease of the activity of the enzyme may between 10% and 100%, such as 20%, 30%, 40%, 50%, 60%, 70%, 80% or 90%; in the case of an increase in activity, the increase may be more than 100%, i.e. 200%, 300%, 500% or more, or may not be measurable as a percentage if the baseline activity of the inhibited enzyme is undetectable.

In a further configuration, the dual specific ligand may bind the substrate of an enzyme/substrate pair, rather than the enzyme. The substrate is therefore unavailable to the enzyme until released from the dual specific ligand through binding of the analyte. The implementations for this configuration are as for the configurations which bind enzyme.

Moreover, the assay may be configured to bind a fluorescent molecule, such as a fluorescein or another fluorophore, in a conformation such that the fluorescence is quenched on binding to the ligand. In this case, binding of the analyte to the ligand will displace the fluorescent molecule, thus producing a signal. Alternatives to fluorescent molecules which are useful include luminescent agents, such as luciferin/luciferase, and chromogenic agents, including agents commonly used in immunoassays such as HRP.

The assay may moreover be configured using a dual specific ligand in the "open" conformation. In this conformation, the dual specific ligand is capable of binding two targets simultaneously. For example, in a first embodiment, the assay may be configured such that the dual specific ligand binds an enzyme and a substrate, where the enzyme has a low affinity for the substrate; and either the enzyme or the substrate is the analyte. When both substrate and enzyme are brought together by the dual specific ligand the interaction between the two is potentiated, leading to an enhanced signal.

Alternatively, the dual specific ligand may bind a fluorescent molecule, as above, which is quenched by the binding of the analyte. In this embodiment, therefore, fluorescence is detectable in the absence of analyte, but is quenched in the presence thereof.

The basic implementation of such an assay is as provided above for closed conformation assays.

Therapeutic and prophylactic uses of dual-specific ligands prepared according to the disclosure involve the administration of ligands selected according to the invention to a recipient mammal, such as a human. Dual-specificity can allow antibodies to bind to multimeric antigen with great avidity. Dual-specific antibodies can allow the crosslinking of two antigens, for example in recruiting cytotoxic T-cells to mediate the killing of tumour cell lines.

Substantially pure antibodies or binding proteins thereof of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected polypeptides may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

The selected antibodies or binding proteins thereof of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J. Immunol., 138: 179).

Generally, the present selected antibodies will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The selected polypeptides of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected polypeptides according to the present invention having different specificities, such as polypeptides selected using different target ligands, whether or not they are pooled prior to administration.

The route of administration of pharmaceutical compositions according to the invention may be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The selected polypeptides of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present selected polypeptides or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected antibody, receptor (e.g. a T-cell receptor) or binding protein thereof *per* kilogram of body weight, with doses of 0.05 to 2.0 mg/kg/dose being more commonly used. For prophylactic applications, compositions containing the present selected polypeptides or cocktails thereof may also be administered in similar or slightly lower dosages.

A composition containing a selected polypeptide may be utilised in prophylactic and therapeutic settings to aid in the alteration, inactivation, killing or removal of a select target cell population in a mammal. In addition, the selected repertoires of polypeptides described herein may be used extracorporeally or *in vitro* selectively to kill, deplete or otherwise effectively remove a target cell population from a heterogeneous collection of cells. Blood from a mammal may be combined extracorporeally with the selected antibodies, cell-surface receptors or binding proteins thereof whereby the undesired cells are killed or otherwise removed from the blood for return to the mammal in accordance with standard techniques.

The invention is further described, for the purposes of illustration only, in the following examples.

### Example 1. Selection of a dual specific seFv antibody (K8) directed against human serum albumin (HSA) and β-galactosidase (β -gal)

This example explains a method for making a dual specific antibody directed against β-gal and HSA in which a repertoire of V_{κ} variable domains linked to a germline (dummy) V_{H} domain is selected for binding to (β-gal and a repertoire of V_{H} variable domains linked to a germline (dummy) V_{κ} domain is selected for binding to HSA. The selected variable V_{H} HSA and V_{κ} β-gal domains are then combined and the antibodies selected for binding to β-gal and HSA.

Four human phage antibody libraries were used in this experiment.

| | | |
|---|---|---|
| Library 1 | Germline V_{κ}/DVT V_{H} | 8.46 x 10⁷ |
| Library 2 | Germline V_{κ}/NNK V_{H} | 9.64 x 10⁷ |
| Library 3 | Germline V_{H}/DVT V_{κ} | 1.47 x 10⁸ |
| Library 4 | Germline V_{H}/NNK V_{κ} | 1.45 x 10⁸ |

All libraries are based on a single human framework for V_{H} (V3-23/DP47 and J_{H}4b) and V_{κ} (O12/02/DPK9 and J_{κ}1) with side chain diversity incorporated in complementarity determining regions (CDR2 and CDR3).
Library 1 and Library 2 contain a dummy V_{κ} sequence, whereas the sequence of V_{H} is diversified at positions H50, H52, H52a, H53, H55, H56, H58, H95, H96, H97 and H98 (DVT or NNK encoded, respectively) (Figure 1). Library 3 and Library 4 contain a dummy V_{H} sequence, whereas the sequence of V_{κ} is diversified at positions L50, L53, L91, L92, L93, L94 and L96 (DVT or NNK encoded, respectively) (Figure 1). The libraries are in phagemid pIT2/ScFv format (Figure 2) and have been preselected for binding to generic ligands, Protein A and Protein L, so that the majority of clones in the unselected libraries are functional. The sizes of the libraries shown above correspond to the sizes after preselection. Library 1 and Library 2 were mixed prior to selections on antigen to yield a single V_{H}/dummy V_{κ} library and Library 3 and Library 4 were mixed to form a single V_{κ}/dummy V_{H} library.

Three rounds of selections were performed on β-gal using V_{κ}/dummy V_{H} library and three rounds of selections were performed on HSA using V_{H}/dummy V_{κ} library. In the case of β-gal the phage titres went up from 1.1 x 10⁶ in the first round to 2.0 x 10⁸ in the third round. In the case of HSA the phage titres went up from 2 x 10⁴ in the first round to 1.4 × 10⁹ in the third round. The selections were performed as described by Griffith *et al.,* (1993), except that KM13 helper phage (which contains a pIII protein with a protease cleavage site between the D2 and D3 domains) was used and phage were eluted with 1 mg/ml trypsin in PBS. The addition of trypsin cleaves the pIII proteins derived from the helper phage (but not those from the phagemid) and elutes bound scFv-phage fusions by cleavage in the c-myc tag (Figure 2), thereby providing a further enrichment for phages expressing functional scFvs and a corresponding reduction in background (Kristensen & Winter, 1998). Selections were performed using immunotubes coated with either HSA or β -gal at 100µg/ml concentration.

To check for binding, 24 colonies from the third round of each selection were screened by monoclonal phage ELISA. Phage particles were produced as described by Harrison *et al.,* (1996). 96-well ELISA plates were coated with 100µl of HSA or β-gal at 10µg/ml concentration in PBS overnight at 4°C. A standard ELISA protocol was followed (Hoogenboom *et al.,* 1991) using detection of bound phage with anti-M13-HRP conjugate. A selection of clones gave ELISA signals of greater than 1.0 with 50µl supernatant (data not shown).

Next, DNA preps were made from V_{H}/dummy V_{κ} library selected on HSA and from V_{κ}/dummy V_{H} library selected on β-gal using the QIAprep Spin Miniprep kit (Qiagen). To access most of the diversity, DNA preps were made from each of the three rounds of selections and then pulled together for each of the antigens. DNA preps were then digested with Sal*I*/Not*I* overnight at 37°C. Following gel purification of the fragments, V_{κ} chains from the V_{κ}/dummy V_{H} library selected on β-gal were ligated in place of a dummy V_{κ} chain of the V_{H}/dummy V_{κ} library selected on HSA creating a library of 3.3 x 10⁹ clones.

This library was then either selected on HSA (first round) and β-gal (second round), HSA/β-gal selection, or on β-gal (first round) and HSA (second round), β-gal/HSA selection. Selections were performed as described above. In each case after the second round 48 clones were tested for binding to HSA and β-gal by the monoclonal phage ELISA (as described above) and by ELISA of the soluble scFv fragments. Soluble antibody fragments were produced as described by Harrison *et al.,* (1996), and standard ELISA protocol was followed (Hoogenboom *et al.,* 1991), except that 2% Tween/PBS was used as a blocking buffer and bound scFvs were detected with Protein L-HRP. Three clones (E4, E5 and E8) from the HSA/β-gal selection and two clones (K8 and K10) from the β-gal/HSA selection were able to bind both antigens (data not shown). scFvs from these clones were PCR amplified and sequenced as described by Ignatovich *et al.,* (1999) using the primers LMB3 and pHENseq (Table 1). Sequence analysis revealed that all clones were identical. Therefore, only one clone encoding a dual specific antibody (K8) was chosen for further work (Figure 3).

### Example 2. Characterisation of the binding properties of the K8 antibody.

Firstly, the binding properties of the K8 antibody were characterised by the monoclonal phage ELISA. A 96-well plate was coated with 100µl of HSA and β-gal alongside with alkaline phosphatase (APS), bovine serum albumin (BSA), peanut agglutinin, lysozyme and cytochrome c (to check for cross-reactivity) at 10µg/ml concentration in PBS overnight at 4°C. The phagemid from K8 clone was rescued with KM13 as described by Harrison *et al.,* (1996) and the supernatant (50µl) containing phage assayed directly. A standard ELISA protocol was followed (Hoogenboom *et al.,* 1991) using detection of bound phage with anti-M13-HRP conjugate. The dual specific K8 antibody was found to bind to HSA and β-gal when displayed on the surface of the phage with absorbance signals greater than 1.0 (Figure 4). Strong binding to BSA was also observed (Figure 4). Since HSA and BSA are 76% homologous on the amino acid level, it is not surprising that K8 antibody recognised both of these structurally related proteins. No cross-reactivity with other proteins was detected (Figure 4).

Secondly, the binding properties of the K8 antibody were tested in a soluble scFv ELISA. Production of the soluble scFv fragment was induced by IPTG as described by Harrison *et al.,* (1996). To determine the expression levels of K8 scFv, the soluble antibody fragments were purified from the supernatant of 50ml inductions using Protein A-Sepharose columns as described by Harlow & Lane (1988). OD₂₈₀ was then measured and the protein concentration calculated as described by Sambrook *et al.,* (1989). K8 scFv was produced in supernatant at 19mg/1.

A soluble scFv ELISA was then performed using known concentrations of the K8 antibody fragment. A 96-well plate was coated with 100µl of HSA, BSA and β-gal at 10µg/ml and 100µl of Protein A at 1µg/ml concentration. 50µl of the serial dilutions of the K8 scFv was applied and the bound antibody fragments were detected with Protein L-HRP. ELISA results confirmed the dual specific nature of the K8 antibody (Figure 5).

To confirm that binding to β-gal is determined by the V_{κ} domain and binding to HSA/BSA by the V_{H} domain of the K8 scFv antibody, the V_{κ} domain was cut out from K8 scFv DNA by Sal*I*/Not*I* digestion and ligated into a Sal*I*/Not*I* digested pIT2 vector containing dummy V_{H} chain (Figures 1 and 2). Binding characteristics of the resulting clone K8V_{κ}/dummy V_{H} were analysed by soluble scFv ELISA. Production of the soluble scFv fragments was induced by IPTG as described by Harrison *et al.,* (1996) and the supernatant (50µ) containing scFvs assayed directly. Soluble scFv ELISA was performed as described in Example 1 and the bound scFvs were detected with Protein L-HRP. The ELISA results revealed that this clone was still able to bind β-gal, whereas binding to BSA was abolished (Figure 6).

### Example 3. Creation and characterisation of dual specific scFv antibodies (K8V_{κ}/V_{H}2 and K8V_{κ}/V_{H}4) directed against APS and β-gal and of a dual specific scFv antibody (K8V_{κ}/VHC11) directed against BCL10 protein and β -gal.

This example describes a method for making dual specific scFv antibodies (K8V_{κ}/V_{H}2 and K8V_{κ}/V_{H}4) directed against APS and β-gal and a dual specific scFv antibody (K8V_{κ}/V_{H}C11) directed against BCL10 protein and β-gal, whereby a repertoire of V_{H} variable domains linked to a germline (dummy) V_{κ} domain is first selected for binding to APS and BCL10 protein. The selected individual V_{H} domains (V_{H}2, V_{H}4 and V_{H}C11) are then combined with an individual β-gal binding V_{κ} domain (from K8 scFv, Examples 1 and 2) and antibodies are tested for dual specificity.

A V_{H}/dummy V_{κ} scFv library described in Example 1 was used to perform three rounds of selections on APS and two rounds of selections BCL10 protein. BCL10 protein is involved in the regulation of apoptosis and mutant forms of this protein are found in multiple tumour types, indicating that BCL10 may be commonly involved in the pathogenesis of human cancer (Willis *et al.,* 1999).

In the case of APS the phage titres went up from 2.8 x 10⁵ in the first round to 8.0 x 10⁸ in the third round. In the case of BCL10 the phage titres went up from 1.8 x 10⁵ in the first round to 9.2 x 10⁷ in the second round. The selections were performed as described in Example 1 using immunotubes coated with either APS or BCL10 at 100µg/ml concentration.

To check for binding, 24 colonies from the third round of APS selections and 48 colonies from the second round of the BCL10 selections were screened by soluble scFv ELISA. A 96-well plate was coated with 100µl of APS, BCL10, BSA, HSA and β-gal at 10µg/ml concentration in PBS overnight at 4°C. Production of the soluble scFv fragments was induced by IPTG as described by Harrison *et al.,* (1996) and the supernatant (50µl) containing scFvs assayed directly. Soluble scFv ELISA was performed as described in Example 1 and the bound scFvs were detected with Protein L-HRP. Two clones (V_{H}2 and V_{H}4) were found to bind APS and one clone (V_{H}C11) was specific for BCL10 (, 7). No cross-reactivity with other proteins was observed.

To create dual specific antibodies each of these clones was digested with Sal*I*/Not*I* to remove dummy V_{κ} chains and a Swl*I*/Not*I* fragment containing β-gal binding V_{κ} domain from K8 scFv was ligated instead. The binding characteristics of the produced clones (K8V_{κ}/V_{H}2, K8V_{κ}/V_{H}4 and K8V_{K}/V_{H}C11) were tested in a soluble scFv ELISA as described above. All clones were found to be dual specific without any cross-reactivity with other proteins (Figure 8).

### Example 4. Creation and characterisation of single V_{H} domain antibodies (V_{H}2sd and V_{H}4sd) directed against APS.

This example demonstrates that V_{H}2 and V_{H}4 variable domains directed against APS (described in Example 3) can bind this antigen in the absence of a complementary variable domain.

DNA preps of the scFv clones V_{H}2 and V_{H}4 (described in Example 3) were digested with Nco*I*/Xho*I* to cut out the V_{H} domains (Figure 2). These domains were then ligated into a Nco*I*/Xho*I* digested pIT1 vector (Figure 2) to create V_{H} single domain fusion with gene III.

The binding characteristics of the produced clones (V_{H}2sd and V_{H}4sd) were then tested by monoclonal phage ELISA. Phage particles were produced as described by Harrison *et al.,* (1996). 96-well ELISA plates were coated with 100µl of APS, BSA, HSA, β-gal, ubiquitin, α-amylase and myosin at 10µg/ml concentration in PBS overnight at 4°C. A standard ELISA protocol was followed (Hoogenboom *et al.,* 1991) using detection of bound phage with anti-M13-HRP conjugate. ELISA results demonstrated that V_{H} single domains specifically recognised APS when displayed on the surface of the filamentous bacteriophage (Figure 9). The ELISA of soluble V_{H}2sd and V_{H}4sd gave the same results as the phage ELISA, indicating that these single domains are also able to recognise APS as soluble fragments (Figure 10).

### Example 5. Selection of single V_{H} domain antibodies directed against APS and single V_{κ} domain antibodies directed against β-gal from a repertoire of single antibody domains.

This example describes a method for making single V_{H} domain antibodies directed against APS and single V_{κ} domain antibodies directed against β-gal by selecting repertoires of virgin single antibody variable domains for binding to these antigens in the absence of the complementary variable domains.

Two human phage antibody libraries were used in this experiment.

| | | |
|---|---|---|
| Library 5 | NNK V_{H} single domain | 4.08 x 10⁸ |
| Library 6 | NNK V_{κ} single domain | 2.88 x 10⁸ |

The libraries are based on a single human framework for V_{H} (V3-23/DP47 and J_{H}4b) and V_{κ} (O12/O2/DPK9 and J_{κ}1) with side chain diversity incorporated in complementarity determining regions (CDR2 and CDR3). V_{H} sequence in Library 5 (complementary V_{κ} variable domain being absent) is diversified at positions H50, H52, H52a, H53, H55, H56, H58, H95, H96, H97 and H98 (NNK encoded). V_{κ} sequence in Library 6 (complementary V_{H} variable domain being absent) is diversified at positions L50, L53, L91, L92, L93, L94 and L96 (NNK encoded) (Figure 1). The libraries are in phagemid pIT1/single variable domain format (Figure 2).

Two rounds of selections were performed on APS and β-gal using Library 5 and Library 6, respectively. In the case of APS the phage titres went up from 9.2 x 10⁵ in the first round to 1.1 x 10⁸ in the second round. In the case of β-gal the phage titres went up from 2.0 x 10⁶ in the first round to 1.6 x 10⁸ in the second round. The selections were performed as described in Example 1 using immunotubes coated with either APS or β -gal at 100µg/ml concentration.

After second round 48 clones from each selection were tested for binding to their respective antigens in a soluble single domain ELISA. 96-well plates were coated with 100µl of 10µg/ml APS and BSA (negative control) for screening of the clones selected from Library 5 and with 100µl of 10µg/ml β-gal and BSA (negative control) for screening of the clones selected from Library 6. Production of the soluble V_{κ} and V_{H} single domain fragments was induced by IPTG as described by Harrison *et al.,* (1996) and the supernatant (50µ) containing single domains assayed directly. Soluble single domain ELISA was performed as soluble scFv ELISA described in Example 1 and the bound V_{κ} and V_{H} single domains were detected with Protein L-HRP and Protein A-HRP, respectively. Five V_{H} single domains (V_{H}A10sd, V_{H}A1sd, V_{H}A5sd, V_{H}C5sd and V_{H}C11sd) selected from Library 5 were found to bind APS and one V_{κ} single domain (V_{κ}E5sd) selected from Library 6 was found to bind β-gal. None of the clones cross-reacted with BSA (Figures 3, 11).

### Example 6. Creation and characterisation of the dual specific scFv antibodies (V_{κ}E5/V_{H}2 and V_{K}E5/V_{H}4) directed against APS and β -gal.

This example demonstrates that dual specific scFv antibodies (V_{κ}E5/V_{H}2 and V_{κ}E5/V_{H}4) directed against APS and β-gal could be created by combining V_{κ}E5sd variable domain that was selected for binding to β-gal in the absence of a complementary variable domain (as described in Example 5) with V_{H}2 and V_{H}4 variable domains that were selected for binding to APS in the presence of the complementary variable domains (as described in Example 3).

To create these dual specific antibodies, pIT1 phagemid containing V_{κ}E5sd (Example 5) was digested with Neo*I*/Xho*I* (Figure 2). Nco*I*/Xho*I* fragments containing V_{H} variable domains from clones V_{H}2 and V_{H}4 (Example 3) were then ligated into the phagemid to create scFv clones V_{κ}E5/V_{H}2 and V_{κ}E5/V_{H}4, respectively.

The binding characteristics of the produced clones were tested in a soluble scFv ELISA. A 96-well plate was coated with 100µl of APS, β-gal and BSA (negative control) at 10µg/ml concentration in PBS overnight at 4°C. Production of the soluble scFv fragments was induced by IPTG as described by Harrison *et al.,* (1996) and the supernatant (50µ) containing scFvs assayed directly. Soluble scFv ELISA was performed as described in Example 1 and the bound scFvs were detected with Protein L-HRP. Both V_{κ}E5/V_{H}2 and V_{κ}E5/V_{H}4 clones were found to be dual specific. No cross-reactivity with BSA was detected (Figure 12).

### Example 7. Construction of vectors for converting the existing scFv dual specific antibodies into a Fab format.

### a. Construction of the C_{κ} vector and Ck/gIII vector.

C_{κ} gene was PCR amplified from an individual clone A4 selected from a Fab library (Griffith *et al.,* 1994) using CkBACKNOT as a 5' (back) primer and CKSACFORFL as a 3' (forward) primer (Table 1). 30 cycles of PCR amplification were performed as described by Ignatovich *et al.,* (1997), except that *Pfu* polymerase was used as an enzyme. PCR product was digested with Not*I*/EcoR*I* and ligated into a Not*I*/EcoR*I* digested vector pHEN14V_{κ} (Figure 13) to create a C_{κ} vector (Figure 14).

Gene III was then PCR amplified from pIT2 vector (Figure 2) using G3BACKSAC as a 5' (back) primer and LMB2 as a 3' (forward) primer (Table 1). 30 cycles of PCR amplification were performed as above. PCR product was digested with Sac*I*/EcoR*I* and ligated into a Sac*I*/EcoR*I* digested C_{κ} vector (Figure 14) to create a Ck/gIII phagemid (Figure 15).

### b. Construction of the C_{H} vector.

C_{H} gene was PCR amplified from an individual clone A4 selected from a Fab library (Griffith *et al.,* 1994) using CHBACKNOT as a 5' (back) primer and CHSACFOR as a 3' (forward) primer (Table 1). 30 cycles of PCR amplification were performed as above. PCR product was digested with Not*I*/BglII and ligated into a Not*I*/Bgl*II* digested vector PACYC4V_{H} (Figure 16) to create a C_{H} vector (Figure 17).

### Example 8. Construction of V_{κ}E5/V_{H}2 Fab clone and comparison of its binding properties with the V_{κ}E5/V_{H}2 scFv version (Example 6).

This example demonstrates that the dual specificity of the V_{κ}E5/V_{H}2 scFv antibody is retained when the V_{κ} and V_{H} variable domains are located on different polypeptide chains. Furthermore, the binding of the V_{κ}E5/V_{H}2 Fab clone to β-gal and APS becomes competitive. In contrast, V_{κ}E5/V_{H}2 scFv antibody can bind to both antigens simultaneously.

To create a V_{κ}E5/V_{H}2 Fab, DNA from V_{κ}E5/V_{H}2 scFv clone was digested with Sal*I*/Not*I* and the purified DNA fragment containing V_{κ}E5 variable domain was ligated into a Sal*I*/Not*I* digested C_{κ} vector (Figure 14). Ligation products were used to transform competent *Escherichia coli* TG-1 cells as described by Ignatovich *et al.,* (1997) and the transformants (V_{κ}ES/C_{κ}) were grown on TYE plates containing 1% glucose and 100µg/ml ampicillin.

DNA from V_{κ}E5/V_{H}2 scFv clone was also digested with Sfi*I*/Xho*I* and the purified DNA fragment containing V_{H}2 variable domain was ligated into a Sfi*I*/Xho*I* digested C_{H} vector (Figure 17). Ligation products were used to transform competent *E. coli* TG-1 cells as above and the transformants (V_{H}2/C_{H}) were grown on TYE plates containing 1% glucose and 10µg/ml chloramphenicol.

DNA prep was then made form V_{κ}E5/C_{κ} clone and used to transform V_{H}2/C_{H}-clone as described by Chung *et al.,* (1989). Transformants were grown on TYE plates containing 1% glucose, 100µg/ml ampicillin and 10µg/ml chloramphenicol.

The clone containing both V_{κ}E5/C_{κ} and V_{H}2/C_{H} plasmids was then induced by IPTG to produce soluble V_{κ}E5/V_{H}2 Fab fragments. Inductions were performed as described by Harrison *et al.,* (1996), except that the clone was maintained in the media containing two antibiotics (100µg/ml ampicillin and 10µg/ml chloramphenicol) and after the addition of IPTG the temperature was kept at 25°C overnight.

Binding of soluble V_{κ}E5/V_{H}2 Fabs was tested by ELISA. A 96-well plate was coated with 100µ1 of APS, β-gal and BSA (negative control) at 10µg/ml concentration in PBS overnight at 4°C. Supernatant (50µ) containing Fabs was assayed directly. Soluble Fab ELISA was performed as described in Example 1 and the bound Fabs were detected with Protein A-HRP. ELISA demonstrated the dual specific nature of V_{κ}E5/V_{H}2 Fab (Figure 18).

The produced V_{κ}E5/V_{H}2 Fab was also purified from 50 ml supernatant using Protein A-Sepharose as described by Harlow & Lane (1988) and run on a non-reducing SDS-PAGE gel. Coomassie staining of the gel revealed a band of 50kDa corresponding to a Fab fragment (data not shown).

A competition ELISA was then performed to compare V_{κ}E5/V_{H}2 Fab and V_{κ}E5/V_{H}2 scFv binding properties. A 96-well plate was coated with 100µl of β-gal at 10µg/ml concentration in PBS overnight at 4°C. A dilution of supernatants containing V_{κ}E5/V_{H}2 Fab and V_{κ}E5/V_{H}2 scFv was chosen such that OD 0.2 was achieved upon detection with Protein A-HRP. 50µl of the diluted V_{κ}E5/V_{H}2 Fab and V_{κ}E5/V_{H}2 scFv supernatants were incubated for one hour at room temperature with 36, 72 and 180µmoles of either native APS or APS that was denatured by heating to 70°C for 10 minutes and then chilled immediately on ice. As a negative control, 50µl of the diluted V_{κ}E5/V_{H}2 Fab and V_{κ}E5/V_{H}2 scFv supernatants were subjected to the same incubation with either native or denatured BSA. Following these incubations the mixtures were then put onto a β -gal coated ELISA plate and incubated for another hour. Bound V_{κ}E5/V_{H}2 Fab and V_{κ}E5/V_{H}2 scFv fragments were detected with Protein A-HRP.

ELISA demonstrated that V_{H}2 variable domain recognises denatured form of APS (Figure 19). This result was confirmed by BIAcore experiments when none of the constructs containing V_{H}2 variable domain were able to bind to the APS coated chip (data not shown). ELISA also clearly showed that a very efficient competition was achieved with denatured APS for V_{κ}E5/V_{H}2 Fab fragment, whereas in the case of V_{κ}E5/V_{H}2 scFv binding to β-gal was not affected by competing antigen (Figure 19). This could be explained by the fact that scFv represents a more open structure where V_{κ} and V_{H} variable domains can behave independently. Such freedom could be restricted in a Fab format.

## Claims

1. A dual-specific ligand comprising a first single immunoglobulin variable domain having a binding specificity to a first antigen which is human serum albumin (HSA) and a second complementary immunoglobulin single variable domain having a binding activity to a second antigen, wherein the first or second single variable domain is a V_{H} domain and the other complementary single variable domain is a V_{L} domain.

2. A dual specific ligand of claim 1 which comprises a universal framework, wherein the universal framework comprises a V_{H} framework selected from the group consisting of DP47, DP45 and DP38; and/or the V_{L} framework is DPK9.

3. A dual specific ligand according to claim 1 or 2 wherein the V_{H} and V_{L} are provided by an antibody scFv fragment.

4. A dual-specific ligand according to claim 1 or 2 wherein the V_{H} and V_{L} are provided by an antibody Fab region.

5. An IgG comprising a dual specific ligand of claim 3 or claim 4.

6. A dual-specific ligand according to any one of claims 1 to 5 wherein the variable regions are non-covalently associated.

7. A dual-specific ligand according to any one of claims 1 to 5 wherein the variable regions are covalently associated.

8. A dual-specific ligand according to claim 7 wherein the covalent association is mediated by di-sulphide bonds.

9. A dual specific ligand of according to any preceding claim which comprises the binding site for a specific generic ligand selected from protein A and protein L.

10. Nucleic acid encoding at least a dual-specific ligand according to any preceding claim.

11. A vector comprising nucleic acid according to claim 10.

12. A vector according to claim 11, further comprising components necessary for the expression of a dual-specific ligand.

13. A host cell transfected with a vector according to claim 12.

14. A method for producing a dual-specific ligand of any one of claims 1 to 9 comprising a first single immunoglobulin variable domain having a first binding specificity and a complementary immunoglobulin single variable domain having a second binding specificity, the method comprising the steps of:
a) selecting a first variable domain by its ability to bind to a first epitope,
b) selecting a second variable region by its ability to bind to a second epitope,
c) combining the variable regions; and
d) selecting the dual-specific ligand by its ability to bind to said first and second epitopes.

15. A method according to claim 14 wherein said first variable domain is selected for binding to said first epitope in absence of a complementary variable domain.

16. A method according to claim 14 wherein said first variable domain is selected for binding to said first epitope in the presence of a third complementary variable domain in which said third variable domain is different from said second variable domain.

## Patentansprüche

1. Dual-spezifischer Ligand, der eine erste variable Immunglobulin-Einzeldomäne mit einer Bindungsspezifität gegenüber einem ersten Antigen, das humanes Serumalbumin (HSA) ist, und eine zweite komplementäre variable Immunglobulin-Einzeldomäne mit einer Bindungsaktivität gegenüber einem zweiten Antigen umfasst, wobei die erste oder zweite variable Einzeldomäne eine V_{H}-Domäne ist und die andere komplementäre variable Einzeldomäne eine V_{L}-Domäne ist.

2. Dual-spezifischer Ligand nach Anspruch 1, der ein Universalgerüst umfasst, wobei das Universalgerüst ein V_{H}-Gerüst, ausgewählt aus der Gruppe bestehend aus DP47, DP45 und DP38 umfasst; und/oder das V_{L}-Gerüst DPK9 ist.

3. Dual-spezifischer Ligand nach Anspruch 1 oder 2, wobei V_{H} und V_{L} durch ein Antikörper-scFv-Fragment bereitgestellt werden.

4. Dual-spezifischer Ligand nach Anspruch 1 oder 2, wobei V_{H} und V_{L} durch eine Antikörper-Fab-Region bereitgestellt werden.

5. IgG, das einen dual-spezifischen Liganden nach Anspruch 3 oder Anspruch 4 umfasst.

6. Dual-spezifischer Ligand nach einem der Ansprüche 1 bis 5, wobei die variablen Regionen nicht-kovalent assoziiert sind.

7. Dual-spezifischer Ligand nach einem der Ansprüche 1 bis 5, wobei die variablen Regionen kovalent assoziiert sind.

8. Dual-spezifischer Ligand nach Anspruch 7, wobei die kovalente Assoziation durch Disulfidbindungen vermittelt wird.

9. Dual-spezifischer Ligand nach einem der vorangehenden Ansprüche, der die Bindungsstelle für einen spezifischen generischen Liganden ausgewählt aus Protein A und Protein L umfasst.

10. Nucleinsäure, die wenigstens einen dual-spezifischen Liganden nach einem der vorangehenden Ansprüche codiert.

11. Vektor, der eine Nucleinsäure nach Anspruch 10 umfasst.

12. Vektor nach Anspruch 11, der des Weiteren Komponenten umfasst, die für die Expression eines dual-spezifischen Liganden notwendig sind.

13. Wirtszelle, die mit einem Vektor nach Anspruch 12 transfiziert ist.

14. Verfahren zur Herstellung eines dual-spezifischen Liganden nach einem der Ansprüche 1 bis 9, der eine erste variable Immunglobulin-Einzeldomäne mit einer ersten Bindungsspezifität und eine komplementäre variable Immunglobulin-Einzeldomäne mit einer zweiten Bindungsspezifität umfasst, wobei das Verfahren die folgenden Schritte umfasst:
a) Selektieren einer ersten variablen Domäne nach deren Fähigkeit, an ein erstes Epitop zu binden,
b) Selektieren einer zweiten variablen Region nach deren Fähigkeit, an ein zweites Epitop zu binden,
c) Kombinieren der variablen Regionen; und
d) Selektieren des dual-spezifischen Liganden nach dessen Fähigkeit, an die ersten und zweiten Epitope zu binden.

15. Verfahren nach Anspruch 14, wobei die erste variable Domäne selektiert wird, um an das erste Epitop in Abwesenheit einer komplementären variablen Domäne zu binden.

16. Verfahren nach Anspruch 14, wobei die erste variable Domäne selektiert wird, um an das erste Epitop in Anwesenheit einer dritten komplementären variablen Domäne zu binden, wobei die dritte variable Domäne von der zweiten variablen Domäne verschieden ist.

## Revendications

1. Ligand à double spécificité comprenant un premier domaine variable unique d'immunoglobuline ayant une spécificité de liaison à un premier antigène qui est la sérumalbumine humaine (HSA) et un second domaine variable unique d'immunoglobuline complémentaire ayant une activité de liaison à un second antigène, dans lequel le premier ou le second domaine variable unique est un domaine V_{H} et l'autre domaine variable unique complémentaire est un domaine V_{L}.

2. Ligand à double spécificité selon la revendication 1 qui comprend une ossature universelle, dans lequel l'ossature universelle comprend une ossature V_{H} choisie dans le groupe constitué par DP47, DP45 et DP38 ; et/ou l'ossature V_{L} est DPK9.

3. Ligand à double spécificité selon la revendication 1 ou 2 dans lequel les V_{H} et V_{L} sont fournis par un fragment scFv d'anticorps.

4. Ligand à double spécificité selon la revendication 1 ou 2 dans lequel les V_{H} et V_{L} sont fournis par une région Fab d'anticorps.

5. IgG comprenant un ligand à double spécificité selon la revendication 3 ou la revendication 4.

6. Ligand à double spécificité selon l'une quelconque des revendications 1 à 5 dans lequel les régions variables sont associées par des liaisons non covalentes.

7. Ligand à double spécificité selon l'une quelconque des revendications 1 à 5 dans lequel les régions variables sont associées par des liaisons covalentes.

8. Ligand à double spécificité selon la revendication 7 dans lequel l'association covalente est médiée par des liaisons disulfure.

9. Ligand à double spécificité selon l'une quelconque des revendications précédentes qui comprend le site de liaison pour un ligand générique spécifique choisi parmi la protéine A et la protéine L.

10. Acide nucléique codant pour au moins le ligand à double spécificité selon l'une quelconque des revendications précédentes.

11. Vecteur comprenant l'acide nucléique selon la revendication 10.

12. Vecteur selon la revendication 11, comprenant en outre les composants nécessaires à l'expression d'un ligand à double spécificité.

13. Cellule hôte transfectée avec un vecteur selon la revendication 12.

14. Procédé de production d'un ligand à double spécificité selon l'une quelconque des revendications 1 à 9 comprenant un premier domaine variable unique d'immunoglobuline ayant une première spécificité de liaison et un domaine variable unique d'immunoglobuline complémentaire ayant une seconde spécificité de liaison, le procédé comprenant les étapes suivantes :
a) choix d'un premier domaine variable en fonction de sa capacité à se lier à un premier épitope ;
b) choix d'un second domaine variable en fonction de sa capacité à se lier à un second épitope ;
c) combinaison des régions variables ; et
d) choix du ligand à double spécificité en fonction de sa capacité à se lier auxdits premier et second épitopes.

15. Procédé selon la revendication 14 dans lequel ledit premier domaine variable est choisi pour sa liaison audit premier épitope en l'absence d'un domaine variable complémentaire.

16. Procédé selon la revendication 14 dans lequel ledit premier domaine variable est choisi pour sa liaison audit premier épitope en présence d'un troisième domaine variable complémentaire dans lequel ledit troisième domaine variable est différent dudit deuxième domaine variable.
